# EUROPEAN PATENT SPECIFICATION

(11) **EP 2 553 105 B1**
(45) Date of publication and mention of the grant of the patent: **17.12.2014**
(21) Application number: 10849117.6
(22) Date of filing: 01.04.2010
(51) Int. Cl.: C12N 15/863, C12N 15/864

(54) **COMPOSITIONS AND METHODS FOR THE PRODUCTION OF RECOMBINANT VIRUS VECTORS**
ZUSAMMENSETZUNGEN UND VERFAHREN ZUR HERSTELLUNG VON REKOMBINANTEN VIRUSVEKTOREN
COMPOSITIONS ET PROCÉDÉS POUR LA PRODUCTION DE VECTEURS DE VIRUS RECOMBINANT

(43) Date of publication of application: 06.02.2013
(73) Proprietor: Xiao, Weidong, Fort Washington, PA 19034 (US)
(72) Inventor: Xiao, Weidong, Fort Washington, PA 19034 (US)
(74) Representative: Rees, Kerry
(86) International application number: PCT/US2010/000985
(87) International publication number: WO 2011/123088

(56) References cited:
- WO-A1-00/17376
- WO-A1-2006/048215
- WO-A2-02/12285
- US-A1- 2002 132 336
- US-B1- 6 387 670
- US-B1- 6 500 623
- B. DONG ET AL: "A concept of eliminating nonhomologous recombination for scalable and safe AAV vector generation for human gene therapy", NUCLEIC ACIDS RESEARCH, vol. 41, no. 13, 1 July 2013 (2013-07-01), pages 6609-6617, XP055074963, ISSN: 0305-1048, DOI: 10.1093/nar/gkt404

## Description

### TECHNICAL FIELD

The technical field generally relates to virology and molecular biology and, in particular, to compositions and methods for the production of recombinant viral vectors.

### BACKGROUND

A recombinant viral vector carrying a foreign DNA insert may be used to deliver genes to cells, where the gene may be expressed to permit production of polypeptides or polynucleotides (e.g., miRNA, RNAi, antisense RNAs) for the treatment or amelioration of diseases or genetic defects in humans or non-human mammals. The foreign DNA insert may also be used for DNA recombination and repair of genes, and no expression would necessary under such circumstances.

Recombinant viral vectors that are commonly used in gene delivery applications are typically defective in one or more genes that are required for viral reproduction, so that the recombinant viral vectors cannot reproduce themselves in patients receiving the treatment. During the production of a recombinant virus, the defective gene or genes are provided *in trans, i.e.,* from a DNA sequence that is physically separated from the recombinant viral genome, so as to permit replication and encapsidation of the recombinant virus. However, since the DNA sequence and the recombinant viral genome are both present in the nucleus of the host cell, recombination events between the DNA sequence and the recombinant viral genome may result in replication-competent viruses that are undesirable for treatment purpose.

Therefore, there still exists a need for methods that are capable of producing recombinant viral vectors that are not contaminated with replication competent viruses and that can be easily scaled-up for industrial production.

### SUMMARY OF THE INVENTION

A method for eliminating or minimizing the formation of replication-competent virus during the production of a replication-deficient recombinant virus vector is disclosed. The replication-deficient recombinant virus vector has a recombinant virus genome with one or more defective viral genes. The method includes infecting a host cell with a carrier virus having a carrier virus genome encoding one or more trans factors or variants thereof, incubating the infected host cell for a desired period of time, and isolating the replication-deficient recombinant virus vector. The carrier virus is a cytoplasmic virus that retains the carrier virus genome in the cytoplasm of the host cell. The one or more trans factors compensate one or more functions of the one or more defective genes in the recombinant virus genome. The one or more trans factors include a structure protein of the replication-deficient recombinant virus. The host cell contains the recombinant viral genome and retains the recombinant viral genome in a nucleus of the host cell.

Also disclosed is a method for producing a replication-deficient recombinant virus vector having a recombinant virus genome with one or more defective viral genes. The method includes infecting a host cell with a carrier virus having a carrier virus genome encoding one or more trans factors or variants thereof, incubating the infected host cell for a desired period of time; and isolating said replication-deficient recombinant virus vector. The carrier virus is a cytoplasmic virus that retains the carrier virus genome in the cytoplasm of the host cell and wherein the one or more trans factors compensate one or more functions of the one or more defective genes in the recombinant virus genome. The host cell contains the recombinant viral genome and retains the recombinant viral genome in a nucleus of the host cell. The replication-deficient recombinant virus vector is selected from the group consisting of recombinant virus vectors from the *Adenoviridae* family, the *Herpesviridae* family, the *Hepadnaviridae* family, and recombinant recombinant parvovirus vectors.

Also disclosed is a method for producing a replication-deficient recombinant AAV vector having a recombinant virus genome that is defective in producing capsid proteins. The method includes infecting a host cell with one or more carrier viruses, incubating the infected host cell for a desired period of time, and isolating the replication-deficient recombinant virus vector. The carrier viruses are cytoplasmic viruses that encode two or more AAV capsid proteins and express the two or more capsid proteins in the cytoplasm of the host cell in a ratio suitable for the production of the replication-deficient AAV vector. The host cell contains the recombinant AAV genome and retains the recombinant AAV genome in a nucleus of the host cell.

Also disclosed is a carrier virus. The carrier virus comprises a capsid and a carrier virus genome that comprises a nucleic acid sequence from a cytoplasmic virus and a nucleotide sequence that encodes two or more AAV structure proteins or variants thereof. The capsid and the nucleic acid sequence from a cytoplasmic virus allow the carrier virus genome to remain in the cytoplasm after entering a host cell. The carrier virus genome expresses the two or more AAV structure proteins or variants thereof in the cytoplasm at a ratio suitable for the production of a recombinant AAV vector that is defective in the two or more AAV structure proteins.

### BRIEF DESCRIPTION OF THE DRAWINGS

**Figure 1** is a diagram showing the prior art method for producing recombinant viral vectors.
**Figure 2** is a diagram showing a method for producing replication-competent virus (RCV) free recombinant virus vectors by sequestering the trans elements and cis elements in different cellular compartments
**Figure 3** is a diagram showing production of recombinant AAV vector using vaccinia virus as a carrier vector for the trans elements.
**Figure 4** is a diagram showing production of recombinant adenovirus vector using vaccinia virus as a carrier vector for the trans elements.
**Figure 5** is a diagram showing production of gutted adenovirus vector using vaccinia virus as a carrier vector for the trans elements.
**Figure 6** is a diagram showing production of recombinant AAV vector using VSV virus as carrier vectors for the trans elements.
**Figure 7** is a map of shuttle plasmid pRB21.
**Figure 8** is a map of shuttle plasmid pRB-repcap.
**Figure 9** is a diagram showing the construction of vvAAV-H1.
**Figure 10** is a picture of a Western blot showing the expression profile of vvAAVvp1, vvAAVvp2 and vvAAVvp3 in HeLa cells using antibodies specific for VP1, VP2 and VP3.
**Figure 11** is a diagram showing the expression profile of Rep and Cap in an AAV production example. First vAd-AAVITR-CMV-GFP infected HeLa cells at an MOI=2, then 5 rVVs (vvAAVrep78, vvAAVrep52,vvAAVvp1, vvAAVvp2,vvAAVvp3) each infected the HeLa cells at an MOI=1 at different time points indicated in the Figure. The HeLa cells were collected after 48 hr rWs transduction. Shown in the Figure is the western blot for cap and rep expression from the harvested cell lysates.

### DETAILED DESCRIPTION

Unless otherwise defined, all technical and scientific terms used herein have the meaning as commonly understood by one of ordinary skill in the art to which this invention belongs. The practice of the present invention employs, unless otherwise indicated, conventional techniques of chemistry, molecular biology, microbiology, recombinant DNA, genetics, virology and immunology. See, *e.g*., Sambrook et al., 2000. Molecular Cloning: A Laboratory Manual, Harlow et al.

A "recombinant viral vector" or a "recombinant virus" refers to a virus produced by recombinant DNA technology. A recombinant virus may be defective in one or more genes that are required for viral reproduction and is, therefore, replication-deficient (*i.e.,* the recombinant virus cannot reproduce itself in a patient receiving the recombinant virus.

A "recombinant viral genome" or "cis element" refers to all the genetic information contained in a recombinant virus. A recombinant viral genome may contain all or a part of a viral genome, wherein the viral genome may be wild-type or may contain point mutations, insertions or deletions. A recombinant viral genome may optionally comprises a transgene operably linked to expression control sequences. The transgene may be flanked by flanking elements. The recombinant viral genome of the invention may be integrated into the host chromosome, or transfected into a host nucleus by physical or chemical methods, or delivered into the host nucleus using a virus vector targeting into the nucleus of the host cells, and is ultimately packaged into a recombinant viral vector.

A "flanking element" or "flanking nucleic acid" is a nucleic acid sequence generally derived from a mammalian virus which, when located in positions flanking a transgene, permits the packaging of the transgene into a recombinant viral vectors. Flanking elements may be the naturally-occurring flanking elements from a mammalian virus which permit the packaging of the recombinant viral vectors, or may be artificial nucleic acid elements, *e.g*. mutated sequences of flanking elements, that have the same or similar packaging function. Flanking elements include, without limitation, the inverted terminal repeats (ITRs) of adeno-associated virus (AAV) or adenovirus (Ad, the long terminal repeats (LTRs) of retrovirus, the "a" or packaging sequence of herpes simplex virus (HSV), as well as any other sequences that are required for packaging from other viruses known in the art.

A "transgene" is a nucleic acid sequence that is to be delivered or transferred to a mammalian cell. A transgene may encode a protein, peptide or polypeptide that is useful as a marker, reporter or therapeutic molecule. A transgene may also encode a protein, polypeptide or peptide that is useful for protein production, diagnostic assays or for any transient or stable gene transfer *in vitro* or *in vivo.* Alternatively, a transgene may encode a functional polynucleotide, such as miRNA, RNAi, antisense RNAs, ribozyme or other regulatory nucleic acids. Transgenes also include DNA sequences that are used to induce DNA recombination and gene repair.

"Expression control sequences" or "regulatory sequences" are nucleic acid sequences that regulate the expression of a gene by being operably linked to the gene of interest. Examples of regulatory sequences include, but are not limited to, appropriate transcription initiation, termination, promoter and enhancer sequences; efficient RNA processing signals such as splicing and polyadenylation signals; sequences that stabilize cytoplasmic mRNA; sequences that enhance translation efficiency (i.e., Kozak consensus sequence); sequences that enhance protein stability; and when desired, sequences that enhance protein secretion. A regulatory sequence may act in cis or trans configuration, or at a distance to control a gene of interest.

A nucleic acid sequence is "operably linked" to another nucleic acid sequence when the former is placed into a functional relationship with the latter. For example, a DNA for a presequence or secretory leader peptide is operably linked to DNA for a polypeptide if it is expressed as a preprotein that participates in the secretion of the hepolypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous and, in the case of a secretory leader, contiguous and in reading phase. However, enhancers do not have to be contiguous. Linking is accomplished by ligation at convenient restriction sites. If such sites do not exist, synthetic oligonucleotide adaptors or linkers are used in accordance with conventional practice.

As used herein, the term "expression cassette" refers to a recombinant DNA molecule containing a desired coding sequence and appropriate nucleic acid sequences necessary for the expression of the operably linked coding sequence in a particular host organism. Nucleic acid sequences necessary for expression in prokaryotes usually include a promoter, an operator (optional), and a ribosome binding site, often along with other sequences. Eukaryotic cells are known to utilize promoters, enhancers, and termination and polyadenylation signals

The term "trans factor" refers to a viral protein that is required for the production of a recombinant virus and is not functionally expressed from the genome of the recombinant virus. A recombinant virus vector may need the help from one or more trans factors to replicate in a host cell. A trans factor can be a wide-type viral protein or a variant of a viral protein. Proteins that can be used as trans factors for the production of recombinant AAV include Rep78, Rep68, Rep 52, rep 40, VP1, VP2 and VP3. Proteins that can be used as trans factors for the production of recombinant parvovirus vectors include NS1, NS2, VP1-3. Proteins that can be used as trans factors for the production of recombinant adenovirus include various adenovirus early gene products and adenovirus later gene products, such as E1a, E1b, E2a, E3, E4, and L1- L5. Proteins that can be used as trans factors for the production of recombinant herpes virus include herpes virus early gene products and herpes virus later gene products (*e.g*., ICP4, ICP27 etc..).

The term "trans factor nucleic acid sequence" refers to a nucleic acid sequence that encodes a trans factor.

The term "trans cassette" refers to an expression cassette that encodes and expresses one or more trans factors.

The term "trans element" refers to a polynucleotide that comprises a trans factor nucleic acid sequence. A trans element may comprises a trans cassette.

The term "carrier virus" refers to a recombinant cytoplasmic virus that carries a trans element in its genome. Examples of carrier viruses include vaccinia carrier virus carrying the herpes ICP4 and/or ICP27 genes for the production of recombinant herpes viral vectors; vaccinia carries virus carrying the AAV vp1 and vp2 genes for the production of recombinant AAV; and VSV carrier viruses carrying the adenovirus E1a and E1b genes for the production of recombinant adenovirus vectors.

The term "cytoplasmic virus" refers to a virus whose nucleic acids predominantly reside in the cytoplasm of the host cells during the infection. Examples of cytoplasmic viruses include, but are not limited to, members of the Poxviridae family, such as variola virus, vaccinia virus, cowpox virus, monkeypox virus, smallpox, pseudocowpox, bovine papular stomatitis virus , tanapox virus, yaba monkey tumor virus molluscum contagiosum virus (MCV), and members of RNA viruses family which does not use a DNA intermediate, such as vesicular stomatitis virus (VSV), Semliki forest virus and Sindbis virus vectors etc.

A "nucleus targeting vector" is a plasmid or viral vector that contains a nuclear anchoring element and is capable of introducing the vector DNA into the nuclei of a cell. Any retrovirus or DNA virus that replicates in the nucleus can be used as nucleus targeting vector. When a viral vector is used as a nucleus targeting vector, it may be referred to as a "virus-based nucleus targeting vector." For example, when a recombinant adenovirus vector carrying a recombinant AAV genome is used as a nucleus targeting vector to introduce the recombinant AAV genome into the nucleus of a host cell, the recombinant adenovirus vector may be referred to as an "adenovirus-based nucleus targeting vector.

The term "nuclear anchoring element" refers to a nuclei acid sequence of a nucleic acid molecule that is retained in a nucleus of a cell, especially in a nucleus of a human cell. This nucleic acid molecule, being a vector and having the nuclear anchoring element, anchors to a nuclear matrix of the nucleus. Examples of a nuclear anchoring element includes, but not limited to, an EBV gene encoding an EBV nuclear protein (*e.g.,* nuclear antigen-1 (EBNA-1)) and an EBV origin (*i.e.,* an EBV replicon, oriP); a papovavirus origin of replication and a papovavirus large T antigen; or a polyomavirus (Py) origin (PyOri) and large T (LT) antigen gene (PyLT) (See *e.g.,* Heffernan and Dennis, 1991, Nucleic Acids Res. 19:85-92).

A "viral regulatory protein" refers to a viral protein that regulates transcription of a viral gene or a host gene.

A "viral structure protein," as used hereinafter, refers to a viral protein that does not regulate transcription. Viral structure proteins are coded by viral structural genes and include nucleocapsid core proteins (such as gag proteins), enzymes (such as pol proteins), and membrane components (such as env proteins). Transcription of viral structural genes is regulated by viral regulatory proteins (such as rep proteins).

A "variant" of a polypeptide is a polypeptide that differs from a native polypeptide in one or more substitutions, deletions, additions and/or insertions, such that the biological activity of the polypeptide is not substantially diminished. In other words, the variant of a protein has similar biological activity or activities as the native protein. In certain embodiments, the biological activity or activities of the variant may be enhanced or unchanged, relative to the native protein, or may be diminished by less than 50%, and preferably less than 20%, relative to the native protein. Preferred variants include those variants in which a small portion (e.g., 1-30 amino acids, preferably 5-15 amino acids) has been removed from the N- and/or C-terminal of the mature protein.

Preferably, a variant polypeptide contains conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged. Amino acid substitutions may generally be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gin, asn, ser, thr; (2) cys, ser, tyr, thr; (3) val, ile, leu, met, ala, phe; (4) lys, arg, his; and (5) phe, tyr, trp, his. A variant may also, or alternatively, contain nonconservative changes. In a preferred embodiment, variant polypeptides differ from a native sequence by substitution, deletion or addition of five amino acids or fewer. Variants may also (or alternatively) be modified by, for example, the deletion or addition of amino acids that have minimal influence on the immunogenicity, secondary structure and hydropathic nature of the polypeptide. Polypeptide variants preferably exhibit at least about 70%, more preferably at least about 90% and most preferably at least about 95% homology to the original polypeptide.

A polypeptide variant also include a polypeptides that is modified from the original polypeptides by either natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched, for example, as a result of ubiquitination, and they may be cyclic, with or without branching. Cyclic, branched, and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination.

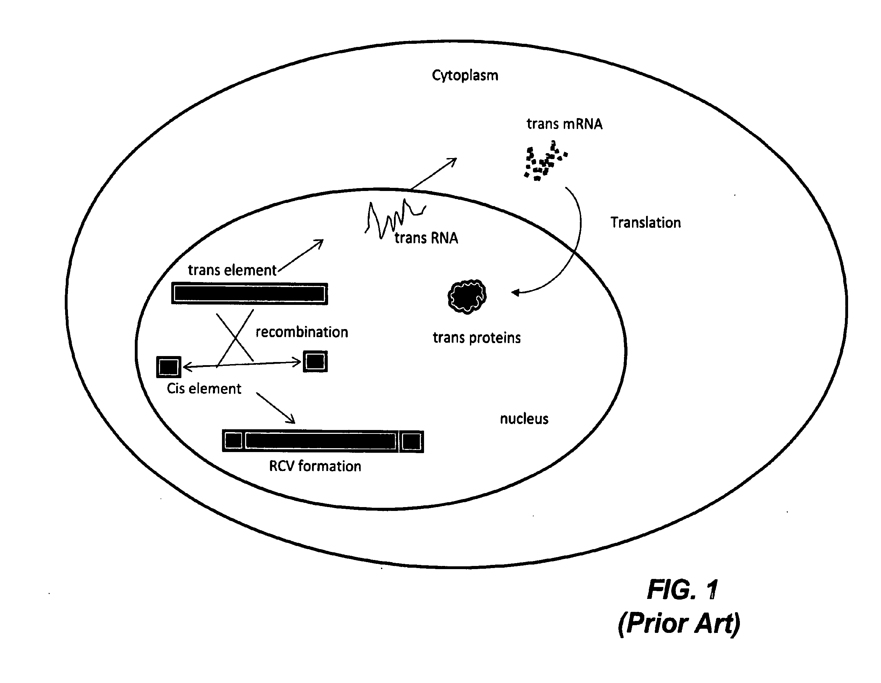

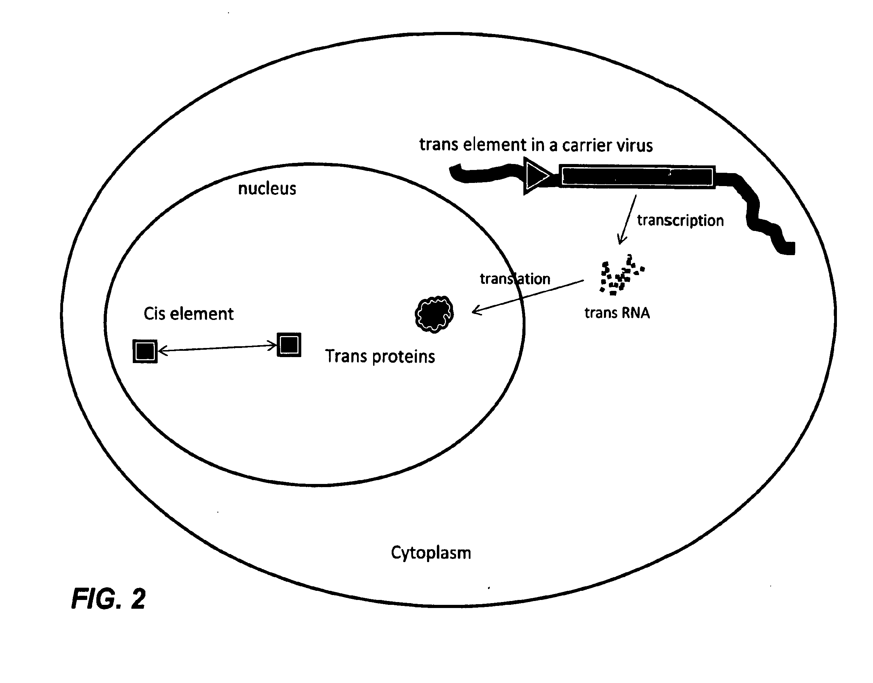

The present invention exploits the properties of cytoplasm born viruses for the creation of novel carrier viruses. The carrier viruses can be used for the manufacture of replication-deficient recombinant viral vectors that are free from replication-competent virus (RCV) particles. Figure 1 shows a prior art system in which both the trans element and the cis element are located in the nucleus of the host and thus allowing the formation of RCV genome by recombination between the trans and cis elements. Figure 2 shows the concept of producing RCV-free recombinant viral vectors by sequestering the trans element and cis element in different cellular compartments. The trans element, which encodes one or more trans factors, resides in the cytoplasm. The cis element, which carries the replication /packaging signals and the transgene, resides in the nucleus of the host cells. The trans factors are transcribed and translated in the cytoplasm and then transported into the nucleus because there are generally nuclear-localization signals in the trans factors. Since the trans elements and the cis elements never meet each other in the same cellular compartment, the possibility of recombination between the two elements, and hence the generation of RCV, is minimized or eliminated.

One aspect of the present invention relates to a method for eliminating or minimizing the formation of replication-competent virus during the production of a replication-deficient recombinant virus having a recombinant viral genome that is defective in one or more viral genes required for viral DNA replication or viral particle production. The method includes the steps of infecting a host cell with at least one or more carrier virus carrying the coding sequence of one or more trans factors or variants thereof that compensate the function of at least one defective gene in the recombinant viral genome, incubating the infected host cell for a desired period of time, and isolating the recombinant virus. The carrier virus is a cytoplasmic virus that retains its viral genome in the cytoplasm of the host cell and expresses the one or more trans factors in the cytoplasm of the host cell. The host cell contains the recombinant viral genome and retains the recombinant viral genome in the nucleus of the host cell.

In certain embodiments, the one or more trans factors include a structure protein of the recombinant viral vector. In other embodiments, the one or more trans factors include only the structure proteins of the recombinant viral vector. In yet other embodiments, the one or more trans factors include both the structure proteins and non-structural proteins of the recombinant viral vector.

The term "replication-competent virus" as used hereinafter, refers to a viral particle that is derived from a replication-deficient recombinant virus and is capable of reproducing infectious virus particles in a host cell in which the replication-deficient recombinant virus cannot reproduce itself. A replication-competent virus typically arises from the homologous or non homologous recombination between the genome of the replication-deficient recombinant virus (*e.g.,* the cis element) and the trans factors genes *(e.g.,* the trans element). The cis element acquires partial or full trans factor functions through the recombination event and gains the ability to reproduce infectious virus particles in the host cell. Replication-competent viruses are often produced in the conventional production system because the cis elements and trans elements are both abundantly present in the nucleus of the host cells. The method of the present invention minimizes or eliminates the likelihood of recombination events between the cis and trans cassettes by retaining the cis and trans cassettes in separate cellular departments.

### The carrier virus

The carrier virus can be any cytoplasmic virus. A cytoplasmic virus retains its genome in the cytoplasm and can be distinguished from viruses that regularly replicate in the nucleus of the host cells. Cytoplasmic carrier virus with trans factor gene(s) incorporated into its genome inherits from the parental wild type virus the property to remain in the cytoplasm after entering a host cell. The carrier virus genome may contain native sequences from a wild-type cytoplamic virus or a modified sequence from a wild-type cytoplamic virus, so long as the genome or the viral capsids retain the function that allows the carrier virus genome to remain in the cytoplasm after entering a host cell. The carrier virus may carry deletions in multiple regions to have more spaces for carrying trans factors. Exogenous genes from different virus may be incorporated into the carrier virus to control the infectivity and tissue tropism of the carrier virus. Chemical conjugation of ligands or non-genetic engineering methods such as formulation may also be used to improve the efficiency of the carrier virus.

In a preferred embodiment, the carrier virus is derived from vaccinia virus. In certain embodiments, multiple vaccinia carrier viruses are used for the production of the recombinant viral vectors and each vaccinia carrier virus carries a trans factor gene. The sequences encoding the trans factors were inserted into the vaccinia virus genome under the control of vaccinia virus promoters or synthetic promoters. Alternatively, the trans factor gene can be controlled by a T7 promoter while T7 RNA polymerase is expressed from a vaccinia virus or the host cells. In other embodiments, one vaccinia carrier virus carries two or more trans factor genes. In a preferred embodiment, a single vaccinia carrier virus carrying all necessary trans factors is used for the production of the recombination viral vectors.

The trans factors that are required for the production of a recombinant viral vector differ depending upon the type of recombinant viral vector and the host cell. The required trans factors for commonly used recombinant viral vectors are known in the art. For example, recombinant AAV vectors typically require the functions of rep and cap for replication and encapsidation, recombinant adenovirus vectors typically require all or part of the functions encoded by E1, E2, E4, L1-L5, pIX and IVa2 genes, alone or in combination, and recombinant herpes virus vectors typically require the functions encoded a large number of genes including UL6, UL18, UL35, UL38 and the major capsid protein UL19, ICP0, RS1(ICP4) and UL54 (ICP27). In general, the host mammalian cell and the carrier virus together must contribute the necessary replication and encapsidation functions for the particular recombinant viral vectors in order to obtain infectious recombinant viral vectors from the mammalian host cells.

The carrier virus may further include a regulatory sequence that regulates the expression of the trans factor nucleic acid sequence or other nucleic acid sequences in the carrier virus. Since the carrier virus resides in the cytoplasm, the regulatory elements for controlling the expression of the trans factor nucleic acid sequence need to be functional in cytoplasm. For example, when poxviruses are used as carrier viruses, cytoplasmic promoters will have to be used to control gene expression. In addition, RNA splicing is lacking for cytoplasmic viruses, different templates will have to be used for gene expression if the proteins are translated from the spliced RNA templates.

The regulatory sequence may comprise a constitutive promoter and/or enhancer. Examples of such promoter/enhancers include, but are not limited to, vaccinia virus p7.5 promoter, vaccinia virus PL11 promoter, I1L promoter (Schmitt and Stunnenberg, 1988, J. Virol. 62, 1889-1897), A10L promoter (Wittek et al., 1984, J. Virol. 49, 371-378), J3R (Senkevich et al., 1997, Virology 233, 19-42), E4L promoter (Ahn et al., 1990, Mol. Cell. Biol. 10, 5433-5441), F 17R promoter (Wittek et al. 1984, Nucl. Acids Res. 12, 4835-4848), A1L promoter (Keck et al., 1990, Cell 61, 801-809),and cowpox A-type inclusion (ATI) late promoter.

The regulatory sequence may also comprise an inducible promoter and/or enhancer. Examples of cytoplasmic inducible promoters include, but are not limited to, the hybrid vaccinia/T7 system, in which a bacteriophage T7 RNA polymerase is used to drive transcription from a T7 promoter (Elroy-Stein et al., 1989, Proc. Nat. Acad. Sci. U.S.A. 86, 6126-6130, and Ward et al., 1995, Proc. Nat. Acad. Sci. U.S.A. 92, 6773-6777). Since T7 RNA polymerase can be expressed the host nucleus, many conventional inducible promoters can be used as Tet-on and Tet-off system, MMTV inducible promoter and others.

In certain embodiments, each protein or polypeptide required for recombinant viral vector production is encoded by a nucleic acid whose expression is regulated by its own cytoplasmic promoter and transcription stop signal, as well as optional sequences such as enhancers. In another embodiment, a trans factor nucleic acid sequence is transcribed to a single transcript that encodes more than one protein or polypeptide required for recombinant viral vector function. In this case, an internal ribosome entry site (IRES) may be placed between the coding sequences of each of the individual proteins or polypeptide to permit subsequent translation of the polycistronic mRNA.

In one embodiment, the carrier vector comprises a trans factor nucleic acid sequence for the production of recombinant AAV. The carrier viruses can be a single virus, a mixture of several viruses of the same type (e.g., several vaccinia carrier viruses each carrying a different trans factors), or a mixture of different types of viruses (e.g., a vaccinia virus carrying a VP1 and a VSV carrying Rep78). In a preferred embodiment, the carrier viruses are vaccinia viruses. In a more preferred embodiment, a single vacinnia carrier virus carrying the AAV rep78, rep 52, rep68, rep 40, vp1, vp2 and vp3 genes is used for the production of recombinant AAV. In another preferred embodiment, a single vacinnia carrier virus carrying the AAV rep78, rep 52, vp1, vp2 and vp3 genes is used for the production of recombinant AAV. In another preferred embodiment, a single vacinnia carrier virus carrying the AAV rep68, rep 52, vp1, vp2 and vp3 genes is used for the production of recombinant AAV. In yet another preferred embodiment, a single vacinnia carrier virus carrying the AAV rep78, rep 52, rep68, rep 40, vp1 and vp2 genes is used for the production of recombinant AAV.

In wild type AAV virus, three capsid genes vp1, vp2 and vp3 overlap each other. A single P40 promoter allows all three capsid proteins to be expressed at a ratio of 1:1:10, which complement with rAAV production. For the production of recombinant AAV vectors, desired ratio of VP1:VP2:VP3 is in the range of about 1:1:1 to about 1:1:100, preferably in the range of about 1:1:2 to about 1:1:50, more preferably in the range of about 1:1:5 to about 1:1:20. Although the desired ratio of VP1:VP2 is 1:1, the ratio range of VP1:VP2 could vary from 1:50 to 50:1. In addition, optimal ratio of Rep78 and Rep52 can be controlled as well. Desired Rep78:Rep52 ratio is in the range of 2:1 to 1:200, preferably in the range of 1:1 to 1:50.

Applicant has found surprisingly that vp2 gene with acg as start codon in a vaccinia carrier virus under the control of p7.5 promoter was able to express a desired ratio of VP2 and VP3 for the production of recombinant AAV. Similarly, vp1 gene with native ATG starting codon in a vaccinia carrier virus was able to express a VP1 and VP3 for the production of recombinant AAV. A mutation of VP1 ATG starting codon to a weak initiation codon like ACG will also help achieve correct ratio of VP1:VP2:VP3 for AAV production. Change of VP2 starting codon to ATG will have an effect on VP2 and VP3 expression as well. Vaccinia carrier virus with both vp1 gene and vp2 gene easily achieved the optimal ratio of VP1, VP2 and VP3 for rAAV production. The desired VP1:VP2:VP3 and/or Rep78:Rep52 ratio can also be obtained by infecting the host cell with multiple viruses at different MOIs (e.g., a first vaccinia virus carrying the vp1 gene at MOI of 1 and a second vaccinia virus carrying the vp2 gene at another MOI).

One advantage of using vaccinia carrier virus carrying one or more of AAV rep78, rep 52, rep68, rep 40, vp1, vp2 and vp3 genes, is that the carrier viruses can be stably maintained and produced. When homologous sequences like vp1 and vp2 are incorporated into a single carrier virus, it is optimal to place them further away in the carrier virus to increase the carrier virus stability. For example, vp1 and rep78 are placed at tk gene locus and vp2 and rep52 are placed at the vp37 locus.

In another preferred embodiment, one vacinnia carrier virus carrying the AAV rep78 and vp1 and one vacinnia carrier virus carrying rep52 and vp2 are used for the production of recombinant AAV. Under this condition, there are no homologous sequences (i.e rep78 vs rep52 or vp1 vs vp2) in any carrier virus. Carrier virus tends to be more stable when there are no homologous DAN sequences. Permutations of rep and capsid genes can be arranged differently. In yet another preferred embodiment, one vacinnia carrier virus carrying the AAV rep78 and vp2 one vacinnia carrier virus carrying rep52 and vp1 are used for the production of recombinant AAV.

AAV has more than 100 serotypes and many modified capsids and the carrier virus can be easily modified to accommodate the production of each serotype and its variants. For example, one vaccinia carrier virus can be used to carry AAV serotype 2 (AAV2) rep 78 and AAV serotype 9 (AAV9) vp2, another carrier virus can be used carry to AAV2 rep52 and AAV9 vp1 to produced pseudeotyped AAV9 vector. In a different embodiment, one vaccinia carrier virus can be used to carry AAV serotype 2 (AAV2) rep 78 and AAV serotype 1 (AAV1) vp2, another carrier virus can be used carry to AAV2 rep52 and AAV8 vp1 to produced pseudeotyped AAV vector with mixed AAV capsids which including AAV8 VP1, AAV8 VP3, AAV1 VP2 and AAV1 VP3.

It is also possible to express only VP1 and not VP3 from vp1 gene in the carrier vector by mutating the VP3 starting codon to other amino acids. Similar approach can be used to control VP3 expression from vp2 gene.

AAV capsid gene can be modified by DNA shuffling and genetic modification which can readily introduced by carrier viruses.

In another embodiment, the trans factor nucleic acid sequence comprises coding sequences for adenovirus E1a, E1b, E2a, E40RF6 and/or VAI. In another embodiment, the helper functions are encoded by a single polycistronic transcript, and the promoter for the helper functions is a constitutive promoter, such as the vaccinia virus p7.5 promoter. In another embodiment, the carrier vector contains a vaccinia virus backbone and the coding sequences for adenovirus E2A and E4orf6. In a related embodiment, the coding sequences of adenovirus E2A and E4orf6 are separated by an internal ribosome entry site (IRES) and are under the control of a single vaccinia promoter.

In another embodiment, the carrier virus is a VSV. Since VSV has a relatively small genome, multiple VSVs may be needed to express all trans factors required for the production of a recombinant virus vector. In some situations, one or several key trans factors are expressed by VSV virus while other trans factors are expressed conventionally from the nucleus.

In some embodiments, two or more carrier vectors are introduced into the host cell. In other embodiments, a single carrier vector is used for the production of the recombinant virus vector.

The carrier virus can be constructed using methods well known in the art. In certain embodiments, the carrier virus is replication-competent in its native host cells but is replication deficient in the host cell for the recombinant virus vectors. For example, a vaccinia carrier virus is replication-competent in BSC, CV-1, and L cells, but is replication-deficient in the Hela cells which are used for the production of the recombiant virus vector of interest.

In certain embodiments, the carrier viruses are constructed with the vRB12 virus backbone, which lacks the functional vp37 gene. The vRB12-based carrier viruses propagate slowly in host cells that do not provide vp37 function, such as CV-1 cells, and extend the incubation period required for reaching cell lysis. This property can be used for the production of recombinant viral vectors when rapid cell lysis by vaccinia carrier virus is not desirable. For example, infecting Hela cells with a vaccinia carrier virus (at an MOI of 1) typically causes cell lysis at day 2 post infection. Infecting the same cells with a vRB 12-based vaccinia carrier virus (vp37 deficient) would delay the cell lysis to day 3 or day 4 post infection. The extended incubation period provides more time for the production of the recombinant virus vectors in the host cell and increasing the recombinant vector yield. Alternatively, temperature sensitive mutants of vaccinia virus can also be used to control the time of host cell lysis.

Vaccinia virus will not productively infect CHO cells. However, cowpox virus can and if the cowpox host range gene is inserted into a vaccinia carrier virus, then the recombinant vaccinia carrier virus will infect CHO cells. The defect is at the stage of intermediate and late protein synthesis. Vaccinia virus also will not infect some lymphocyte lines and non-vertebrate cell lines. Such modification provides another way to control vaccinia virus replication and optimize the parameters for recombinant viral vector production.

In certain embodiments, one or more carrier vectors and/or carrier viruses may be constructed. The one or more carrier vectors and/or carrier viruses comprise all of the elements required to produce a replication-deficient recombinant viral vectors in a particular host cell or cell line. The number and type of elements that are required will depend upon the particular host cell used and the type of recombinant viral vectors to be produced.

### Helper functions required for recombinant AAV production

The production of recombinant AAV typically requires helper functions from certain adenovirus proteins such as adenovirus E1a, E1b and E2a proteins, and probably adeonvirus E40RF6 and VAI proteins as well. The helper functions may also be provided from the helicase-primase complex of herpes simplex virus (HSV) (e.g., UL5, UL8 and UL52), the major single-stranded DNA binding protein of HSV (UL29), products of all 7 HSV DNA replication genes (UL5, 8, 52, 29, 30, 9 and 42). The helper functions for recombinant AAV may also be provided by chemical or physical agents, including ultraviolet light, cycloheximide, hydroxyurea and various carcinogens. The helper functions may be provided by the host cell, or by a helper virus vector. The helper virus vector can be an adenovirus, an HSV, a cytomegalovirus (CMV), a vaccinia virus or pseudorabies virus (PRV). The helper functions may also be provided by a carrier virus.

### The recombinant viral genome (cis cassette)

The recombinant viral genome, or the "cis element," is resided in the nucleus of the host cell in either integrated form or episomal form. The recombinant viral genome cannot be excised, replicated, and packaged into virions without the trans factor(s) from the carrier virus vector. However, once the carrier virus infects the host cell hosting the recombinant viral genomes, the essential gene products required for replication and packaging of the recombinant viral genome will be expressed from carrier virus DNA in the cytoplasm. Because these essential gene products often have nucleus translocation signal. The mature proteins are then transported to the nucleus and meet the recombinant viral genomes, where the replication and packaging of recombinant viral DNA will take place. In this setting, the carrier vector DNA remains in the cytoplasm and does not meet the recombinant viral genome, which stays in the nuclei of the host cell. Therefore, the possibility of recombination between the recombinant viral vector genomes and carrier vector genomes, which may otherwise lead to the generation of replication competent viral particles, is reduced greatly or completely eliminated.

In certain embodiments, a recombinant viral genome may be inserted into a nucleus targeting vector and introduced into a host cell prior to, or concurrently with, the introduction of a carrier vector. The commonly used nucleus targeting vector include HSV or adenovirus. Lentiviral vector or retroviral vector may also be used to facilitate the integration of the cis cassette into the host chromosome.

In certain embodiments, the nuclear anchoring element is the EBV gene and the EBV origin. It has been shown that the anchoring effect of the EBV gene and the EBV origin is due to a high-affinity matrix attachment of the oriP sequence and an interaction of oriP with the origin binding protein, EBNA-1 (See e.g., Polvino-Bodnar and Schaffer, 1992, Virology 187, 591-603). In another embodiments, sv40 replication origin can also be used increase the copy number of the cis element.

The advantage of having the recombinant viral genome on a nucleus targeting vector is that it eliminates the need to establish a stable cell line that harbors the recombinant viral genome. The nucleus targeting vector would bring the recombinant viral genome into the nucleus of the host cell, thus achieve the goal of separating the recombinant viral genome from the carrier vector, which stays in the cytoplasm of the host cell.

### The host cells

The host cell can be any cell that is infectible with a carrier virus. In certain embodiments, mammalian cell lines may be generated to facilitate the production of recombinant viral vectors. The cell lines stably express (either on an extrachromosomal episome or through integration in the cell's genome) certain trans factors and, in the case of AAV production, helper functions. For example, 293 cells (ATCC CRL-1573). constitutively produce adenoviral E1a and E1b proteins, which may serve as trans factors for the production of recombinant adenovirus or provide helper functions for the production of recombinant AAV. A stably transformed mammalian cell may provide some of the trans factor/helper functions, while other trans factor/helper functions are introduced into the cytoplasm of the host cell by a carrier virus.

Examples of the host cells include, but are not limited to, Hela cells, cos cells, BHK cells, A549 cells, and other primary or transformed T-cells, fibroblast cells, hepatocytes, endothelial cells, megkaryotcyes etc.

In certain embodiments, the host cell is a cell that is not receptive to the recombinant viral vector to be produced in the host cell, so that a host cell cannot be re-infected by the recombinant viral vector released by another lysed host cell. For example, human megakaryocytic leukaemia cell line, MB-02, Mo-7e, and myeloid cell line KG1, (Handa et al., J of Gen. Virol. 2000, 81, 2077-2084.) which are resistant to AAV serotype 2 infection but is susceptive to adenovirus and vaccinia virus infection, may be used as the host cell for the production of recombinant AAV with a vaccinia carrier virus.

In other embodiments, the host cells are cultured in suspension to improve the yield of the recombinant viral vectors. In other embodiments, the host cells are cells grown in an *in vivo* setting, such as hepatocytes in a mouse liver. The cells are infected *in vivo* by one or more carrier viruses and a nuclear target virus carrying a recombinant viral genome. The recombinant viral vectors are produced *in vivo.*

In certain embodiments, the host cell is a stably transformed call that expresses one or more proteins with helper functions or trans factors. In other embodiments, the method further includes introducing into the host cell a nuclear targeting vector containing a recombinant viral vectors genome. In a related embodiment, the nuclear targeting vector is a viral vector. In another embodiment, the nuclear targeting vector is an adenovirus vector. In yet another embodiment, the nuclear targeting vector is an adenovirus vector carrying a recombinant AAV genome.

### The replication-deficient recombinant virus vectors

The method of the present invention can be used for the production of any replication-deficient recombinant virus vector that replicates the viral genome in the nuclei of the host cells. Examples of such replication-deficient recombinant virus vectors include, but are not limited to, recombinant viruses of the *Adenoviridae* family, such as recombinant adenovirus vectors, recombinant viruses of the *Herpesviridae* family, such as herpes simplex virus (HSV) vectors, recombinant viruses of the *Hepadnaviridae* family, such as hepatitis B virus (HBV), recombinant viruses of the *Parvoviridae* family, such as AAV vectors, and any other DNA virus.

In certain embodiments, the replication-deficient recombinant virus vector is a viral vector from the *Parvoviridae* family, which includes viruses in the *Parvovirus* genus, such as LuIII virus and minute virus of mice (MVM), and viruses in the *Dependoviruses* genus, such as AAV. The method includes the steps of infecting a host cell with one or more carrier viruses, incubating the infected host cell for a desired period of time; and isolating the recombinant virus. The recombinant virus genome is introduced into the nucleus of the host cell by infecting the host with a recombinant adenovirus or herpes virus vector that carries the recombinant virus genome prior to the infection of the one or more carrier viruses.

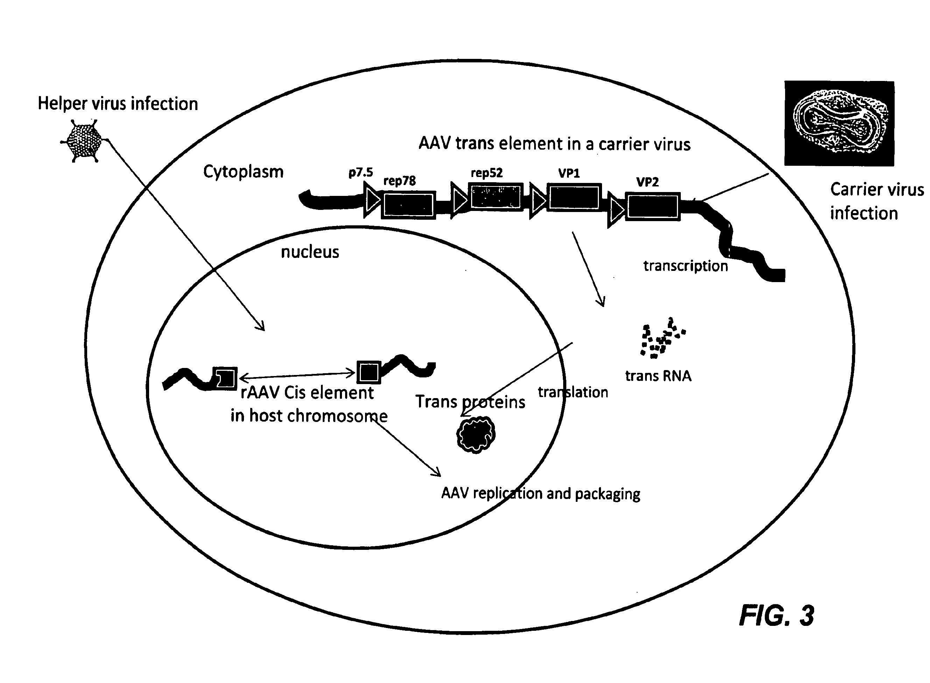

In a preferred embodiment, the replication-deficient recombinant virus vector is an AAV vector, and the one or more carrier viruses carry the coding sequence for a capsid protein of AAV. The one or more carrier viruses can be a single virus, a mixture of several viruses of the same type (e.g., several vaccinia carrier viruses each carrying a different trans factors), or a mixture of different types of viruses (e.g., a vaccinia virus carrying a VP1 and a VSV carrying Rep78). In a preferred embodiment, the carrier viruses are vaccinia viruses. In a more preferred embodiment, a single vacinnia carrier virus carrying the AAV rep78, rep 52, vp1 and vp2 genes is used for the production of the recombinant AAV. **Figure 3** is a diagram showing production of recombinant AAV vector using vaccinia virus as a carrier vector for the trans elements.

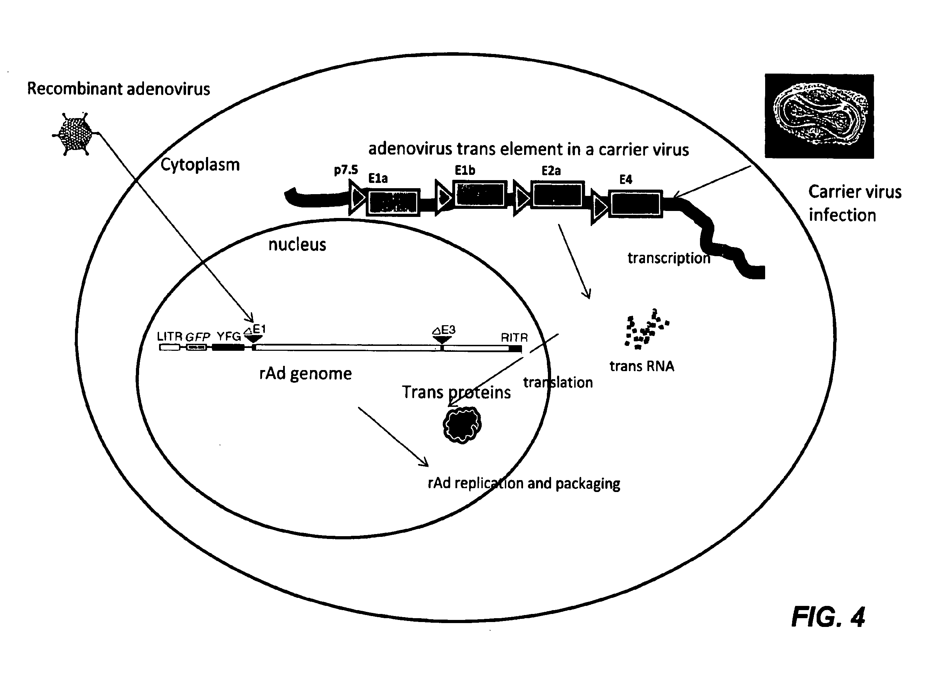

In other embodiments, the replication-deficient recombinant virus vector is an adenovirus vector. Examples of recombinant adenoviral vectors include, but are not limited to, E1-deleted adenoviral vectors, E1- and E2-deleted adenoviral vectors, E1-, E2- and E4-deleted adenoviral vectors, and gutless adenovirus vectors. **Figure 4** is a diagram showing production of recombinant adenovirus vector using vaccinia virus as a carrier vector for the trans elements. Since the trans factors carried by the carrier virus may vary, all known adenovirus vectors can be produced in this way which includes but not limited to E1, E3 deleted adenovirus vectors, E1, E2, E3, and E4 deleted adenovirus vectors and gutless adenovirus vectors.

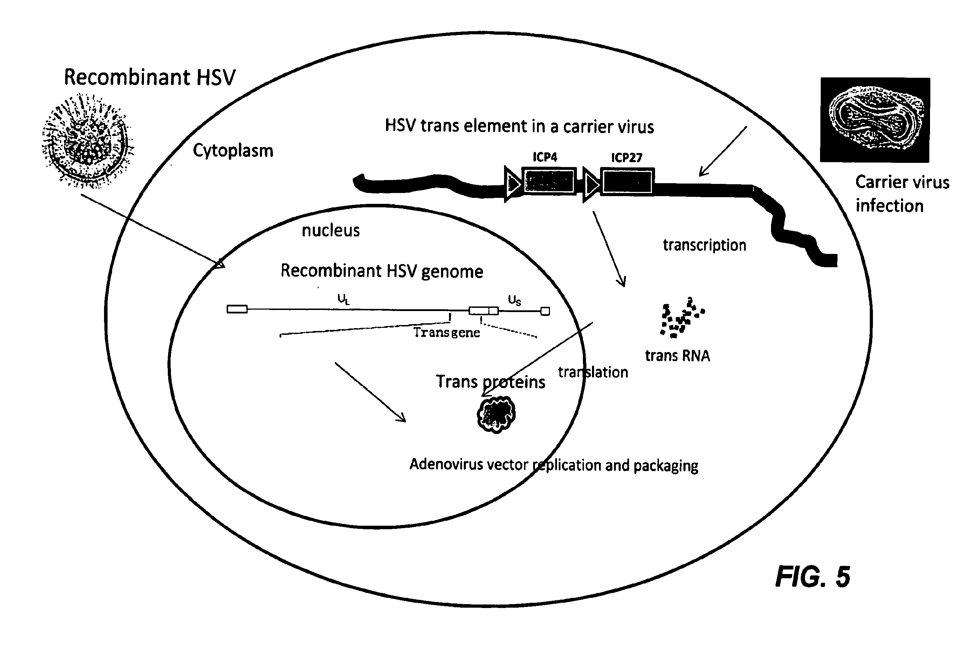

In other embodiments, the replication-deficient recombinant virus vector is a HSV vector. **Figure 5** is a diagram showing production of recombinant HSV vector using vaccinia virus as a carrier vector for the trans elements. In another embodiments, HSV-1 amplicon vectors can be produced by supplied ICP4, ICP27 by a carrier virus and the rest helper genes by cosmids.

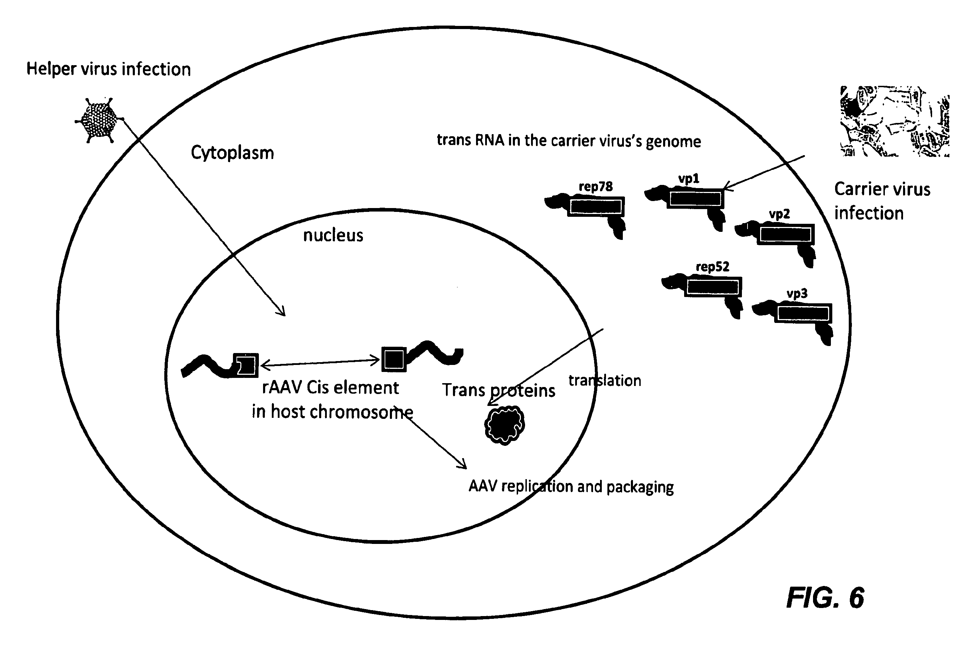

In other embodiments, the replication-deficient recombinant virus vector is an AAV vector. **Figure 6** is a diagram showing production of recombinant AAV vector using VSV as carrier vectors for the trans elements.

### Infection of the host cell with the carrier virus

Infection of the host cells by the carrier virus(s) is carried out under conditions suitable for the particular carrier virus/host cell combination. The infection conditions (e.g., cell density, multiplicity of infection, composition of the infection medium, infection temperature, etc.) can be determined by one skilled in the art based on the host cell type and the characteristics of the viruses used for the infection.

In certain embodiments, the host cells may be transfected or infected with a nucleus targeting vector prior to the infection of the carrier viruses, incubated for a desired period of time to allow the nucleus targeting vector to enter the nuclei of the host cell, and then infected with the carrier virus. The length of the incubation period can be determined experimentally depending on the type of host cell and the type of nucleus targeting vector. In one embodiment, the host cells are first infected with the recombinant adenovirus-based nucleus targeting vector or HSV-based nucleus targeting vector carrying the a recombinant viral genome, and then infected with the carrier virus with the required trans factor(s) after an incubation period of about 6-24 hours, about 8-18 hours, about 10-14 hours, or about 12 hours.

In certain embodiment, the host cells are infected with an adenovirus-based nucleus targeting vector carrying a recombinant AAV genome. The adenovirus-based nucleus targeting vector can be any adenovirus that is capable of providing the helper function required for the production of the recombinant AAV in the host cell. Examples of such adenoviruses include, but are not limited to, first generation adenovirus with deletion in E1a, E1b, 2^{nd} generation or third generation of adenovirus with more deletions. Besides human adenovirus, non human primate adenovirus or adenovirus from other species can also be used.

### Isolation of recombinant virus vectors

Methods for isolation of recombinant viral vectors from host cells are well known in the art. General methods include centrifugation or ultracentrifugation with CsCl gradient or sucrose gradient, or iodixanol gradient, filtration (e.g., using a 0.22µm filter), chromatography or combinations thereof. Methods for purifying recombinant AAV vectors are described, for example, in Current Protocols in Human Genetics, UNIT 12.9 Production of Recombinant Adeno-Associated Viral Vectors by Vivian W. Choi, Aravind Asokan, Rebecca A. Haberman, and Richard Jude Samulski. Methods for purifying recombinant adenovirus vectors are described, for example, in Current Protocols in Human Genetics, UNIT 12.4, Adenoviral Vectors, by Tong-Chuan He,; UNIT 12.13, Helper-Dependent Adenoviral Vectors, by Kazuhiro Oka, Lawrence Chan. Methods for purifying recombinant HSV vectors are described, for example, in Current Protocols in Human Genetics, UNIT 12.11, Construction of Replication-Defective Herpes Simplex Virus Vectors, by William F. Goins, Peggy Marconi, David Krisky, Darren Wolfe, Joseph C. Glorioso, Ramesh Ramakrishnan, and David J. Fink,; UNIT 12.12, Gene Delivery Using Helper Virus-Free HSV-1 Amplicon Vectors, by Cornel Fraefel.

If a vaccinia virus is used as the carrier virus, the vaccinia carrier virus particles can be easily removed from the lysate of host cells by filtration. If the host cells are cultured in a suspension culture, recombinant viruses may be harvested from the supernatant of the culture medium. In certain embodiments, recombinant AAV vectors produced using vaccinia carrier viruses are purified by lysing the host cells, removing large cellular debris by centrifugation and passing the centrifugation supernatant through a 0.22µm filter to remove carrier virus.

In certain embodiments, recombinant AAV vectors prepared by the method of the present invention are isolated using as described column chromatography by Gao et al. (Hum Gene Ther. 2000, 11:2079-2091).

Another aspect of the present invention relates to a replication-deficient recombinant viral vectors produced using the method of the present invention. The recombinant DNA viruses produced with method of the present invention is free from replication-competent viral particles. As used herein a virus that is "free from replication-competent viral particles" refers to a replication-deficient recombinant virus stock that (1) contains no detectable level of replication-competent virus, or (2) contains replication-competent virus at a level that is below a threshold level.

In certain embodiments, the recombinant DNA virus free from replication-competent viral particles is a replication-deficient recombinant AAV stock that contains no detectable replication-competent viral particles or a replication-deficient recombinant AAV stock that contains replication-competent viral particles at a level below one replication-competent viral particle out of every 10¹⁰ recombinant viral particles, every 10" recombinant viral particles, every 10¹² recombinant viral particles, or every 10¹³ recombinant viral particles.

In other embodiments, the recombinant DNA virus free from replication-competent viral particles is a replication-deficient recombinant adenovirus stock that contains no detectable replication-competent viral particles or a replication-deficient recombinant adenovirus stock that contains replication-competent viral particles at a level below one replication-competent'viral particle out of every 10⁹ recombinant viral particles, every 10¹⁰ recombinant viral particles, every 10" recombinant viral particles, or every 10¹² recombinant viral particles.

In other embodiments, the recombinant DNA virus free from replication-competent viral particles is a replication-deficient recombinant HSV stock that contains no detectable replication-competent viral particles or a replication-deficient recombinant HSV stock that contains replication-competent viral particles at a level below one replication-competent viral particle out of every 10⁷ recombinant viral particles, every 10⁸ recombinant viral particles, every 10⁹ recombinant viral particles, or every 10¹⁰ recombinant viral particles.

Methods for detecting. replication-competent recombinant virus particles in a stock of replication-deficient recombinant virus are well established in the field of art. Examples of such methods include, but are not limited to, Southern blot analysis of wild type virus genomes or ELISA/Western blot analysis of viral particles after multiple rounds of amplification under permissive replication conditions for wild type virus. Such methods have been described, for example, by Allen et al. (J. Virol. 1997, 71:6816-6822) (detection of replication-competent AAV particles), Marzio et al. (Vaccine, 2007, 25:2228-2237 (detection of replication-competent adenovirus particles), and Goins et al. (Methods Mol Biol. 2008, 433:97-113 (detection of replication-competent HSV particles).

Another aspect of the present invention relates to a carrier virus for the production of a replication-deficient recombinant viral vectors based on single stranded or double stranded DNA viruses. The carrier virus contains in its genome a cytoplasmic virus backbone and a nucleotide sequence encoding one or more trans factors required for the production of a replication-deficient recombinant virus vector.

In certain embodiments, the nucleotide sequence encoding one or more trans factors encodes a structure protein of the replication-deficient recombinant virus vector.

In other embodiments, the nucleotide sequence encoding one or more trans factors encodes one or more AAV capsid proteins. In one embodiment, the carrier virus is a vaccinia carrier virus carrying one or more nucleotide sequence encoding AAV capsid proteins. In another embodiment, the carrier virus is a vaccinia carrier virus carrying the AAV vp1 and vp2 genes, each gene is controlled by its own promoter.

In other embodiments, the carrier virus is a vaccinia carrier virus carrying one or more nucleotide sequence encoding adenovirus E1a, E1b, E2a and /or E4ORF6 proteins.

In other embodiments, the carrier virus is a vaccinia carrier virus carrying one or more nucleotide sequence encoding HSV ICP4 and ICP27 proteins.

### EXAMPLES

### Example 1. Construction of vaccinia carrier viruses carrying adenovirus genes

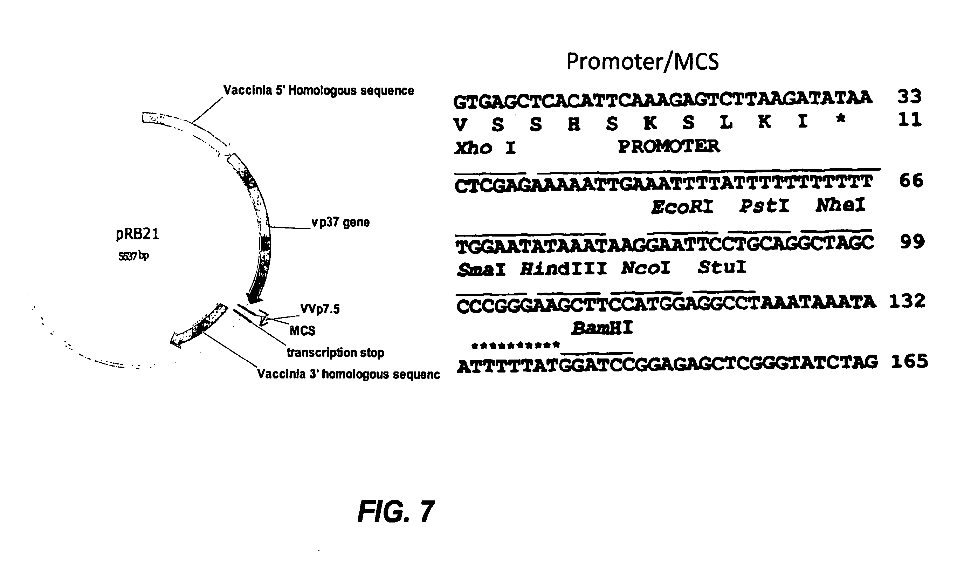

The adenovirus cDNA gene, E1a, E1b, E2a, E4ORF6 are PCR amplified and cloned into plasmid vector pRB21 (Blasco and Moss, Gene, 1995, 158:157-162) in the MCS behind the vaccina virus p7.5 promoter. Figure 7 shows a plasmid map of pRB21. The resulting shuttle plasmids, designed pRB-E1a, pRB-E2b, pRB-E2a, pRB-E4ORF6, are then transfected into BSC cells and infected with vaccinia virus vRB12 (supra). The vRB12 virus lacks the gene encoding protein VP37. pRB21 provides a complete copy of the VP37 gene, thus allowing recombinant vaccinia virus to be selected on the basis of plaque formation. The carrier virus clone which expressed these gene products were then picked and amplified as described by Blasco and Moss (supra).

### Example 2. Construction of vaccinia carrier viruses carrying HSV genes.

The cDNA for HSV ICP4 and ICP27 are PCR amplified and cloned into pRb21 in the MCS behind the p7.5 promoter. The resulting shuttle plasmids pRB21-ICP4 and pRB21-ICP27 were then transfected into BSC cells and infected with vRB12. The carrier virus clone which expressed hepes gene were then picked and amplified as described in Example 1.

### Example 3. Construction of vaccinia carrier viruses with AAV genes

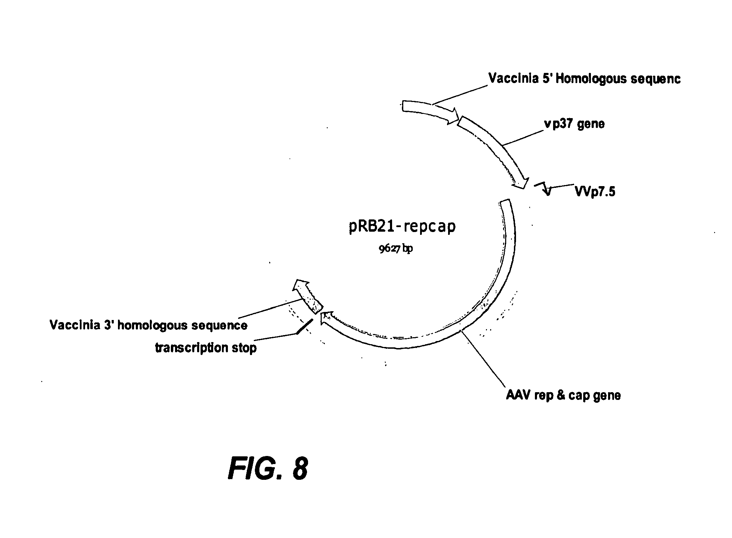

AAV rep and cap gene were PCR amplified from the ATG of rep78 to the stop codon of VP3 and cloned into pRb21 behind the p7.5 promoter **(SEQ ID NO:1).** The resulting shuttle plasmids, designated pRB-repcap **(****Figure 8****),** was then transfected into BSC cells and infected with vRB12. The carrier virus clone which expressed AAV genes were then picked and amplified as described in Example 1. The resulting vaccinia carrier virus is named vvAAVRepcap. Western blot analysis showed that vvAAVrepcap mainly expressed Rep78. There are no cap proteins expressed. It demonstrated that each gene of AAV has to be specially cloned in order to provide proper gene expression.

AAV rep78, rep 68, rep52, rep40, vp1, vp2 and vp3 genes were PCR amplified. The amplified genes were individually cloned into pRb21 behind the p7.5 promoter. The resulting shuttle plasmids, designated pRB-rep78, pRB-rep68, pRB-rep52, pRB-Rep40, pRB-vp1, pRB-vp2 and pRB-vp3 were then transfected into BSC cells and infected with vRB12. The carrier virus clone which expressed the AAV genes were then picked and amplified as described in Example 1. The resulting vaccinia carrier viruses are designated vvAAVrep78, vvAAVrep68, vvAAVrep52, vvAAVRep40, vvAAVvp1, vvAAVvp2 and vvAAVvp3, respectively. The sequences of the trans cassettes in these viruses are shown in **SEQ ID NO:2** (rep78 expression cassette), **SEQ ID NO:3** (rep68 expression cassette), **SEQ ID NO:4** (rep52 expression cassette), **SEQ ID NO:5** (rep40 expression cassette), **SEQ ID NO:6** (vp1 expression cassette), **SEQ ID NO:7** (vp2 expression cassette) and **SEQ ID NO:8** (vp3 expression cassette),

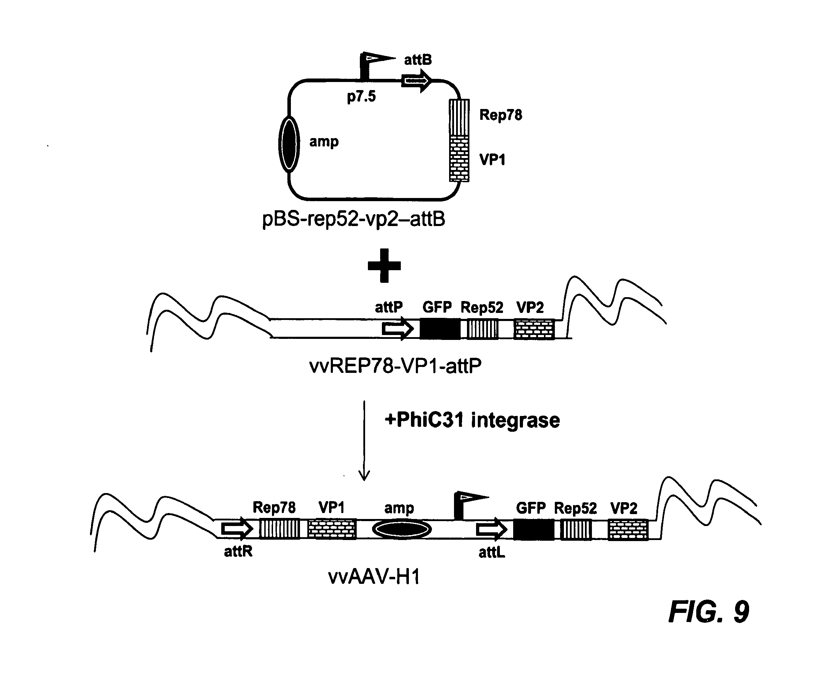

To construct a single vaccinia carrier virus expressing rep78, rep52, VP1, VP2 and VP3 for recombinant AAV production (rep68 and rep40 are not necessary for recombinant AAV production), a vaccinia carrier virus expressing Rep78 and VP1 was constructed first. Briefly, both rep78 and vp1 genes were cloned into pRB21 under their own p7.5 promoters to generate shuttle plasmid pRB=rep78vp1-attP **(****Figure 9****).** As shown in **Figure 9****,** a phage attachment (*attP*) site was also included in the shuttle plasmid. The rescued vaccinia carrier virus was named vvREP78-VP1-attP.

The second shuttle vector was constructed using pBluecript-SK+ as the backbone: Rep52 and VP2 under their own p7.5 promoter were cloned into pBluescript-SK+ along with a phage attachement (*attB*) site. The resulting shuttle plasmid is designated pBS-rep52-vp2-attB. After BSC cells were transfected with pBS-rep52-vp2-attB and expressing plasmid for phiC37 integrase, the cells were infected with vvREP78-VP1-attP, the resulting vaccinia carrier virus, which carries AAV rep78, rep52, vp1 and vp2 genes, is designated as vvAAV-H1 **(****Figure 9****).**

### Example 4. Expression of AAV capsid protein by vaccinia carrier viruses

The rescued carrier viruses were tested for their ability to express the AAV genes carried in the viral genome. Briefly, vvAAVrep78, vvAAVrep68, vvAAVrep52, vvAAVRep40, vvAAVvp1, vvAAVvp2 and vvAAVvp3 were respectively used infect Hela cells at a MOI of 1. The cell lysates were collected at 48 hours post-transfection and antibodies specific for AAV2 capsid protein were used to Western blot detection of capsid proteins.

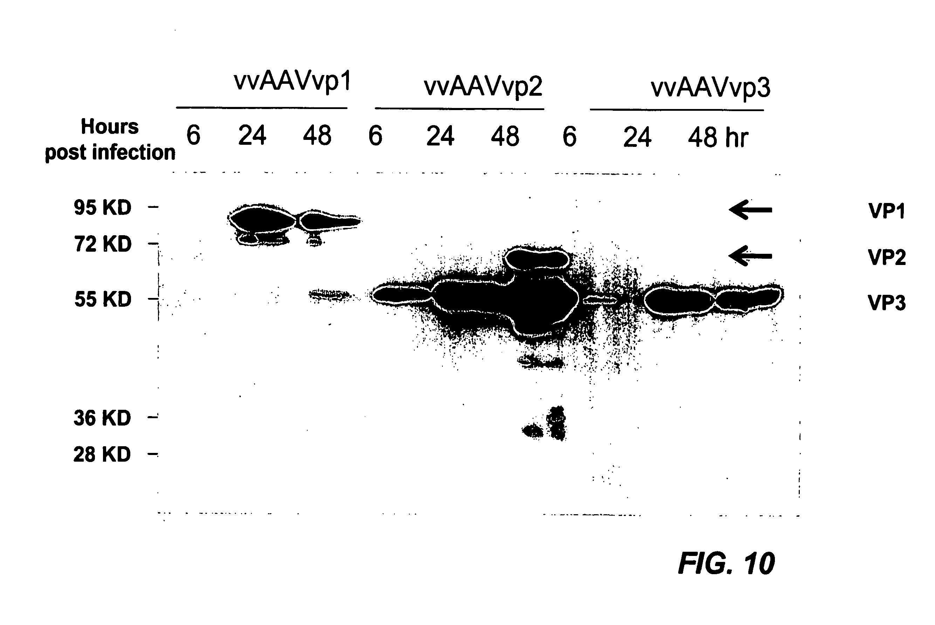

The Western blots revealed vvAAVrep78, vvAAVrep68, vvAAVrep52, vvAAVRep4O expressed rep78, rep68, rep52 and rep40 respectively. vvAAVvp3 expressed only VP3. However, vvAAVvp1 expresses both VP1 and VP3. The expression of VP3 is minor. vvAAVvp2 expresses both VP2 and VP3. VP3 is a major component. This is shown in the **Figure 10****.**

In wild type AAV virus, three capsid genes vp1, vp2 and vp3 overlap each other. A single P40 promoter allows all three capsid proteins to be expressed at a ratio of 1:1:10, which complement with rAAV production. Although the vaccinia carrier virus vvAAVvp1 has the cassette for VP1, VP2 and VP3 under the control of p7.5 promoter, the western blot analysis showed that only VP1 and VP3 were expressed. However, the VP1, VP2, VP3 proteins are expressed at a ratio (1:1:10) suitable for AAV production when vvAAVvp1, vvAAVvp2 infects the HeLa cells at a ratio of 1:1.

### Example 5. Construction of nucleus targeting vectors carrying AAV ITR-CMV-GFP

The recombinant AAV genomes including two copy of AAV ITR and a GFP expression cassette containing a CMV promoter, the GFP gene and a polyadenylation site were cloned into the pShuttle plasmid (Invitrogen, Life Technologies Corp., Carlsbad, California). Adenovirus vector carrying AAV ITR-CMV-GFP were then rescued out with pAdeasy kit (Invitrogen, Life Technologies Corp., Carlsbad, California). and purified as described in Current Protocols in Human Genetics, UNIT 12.4, Adenoviral Vectors, by Tong-Chuan He. The vector was named as vAd-AAVITR-CMV-GFP. It is used to deliver AAVITR-CMV-GFP sequence to the nucleus of the cells.

### Example 6. Production of recombinant AAV using vaccinia carrier viruses and vAd AAVITR-CMV-GFP

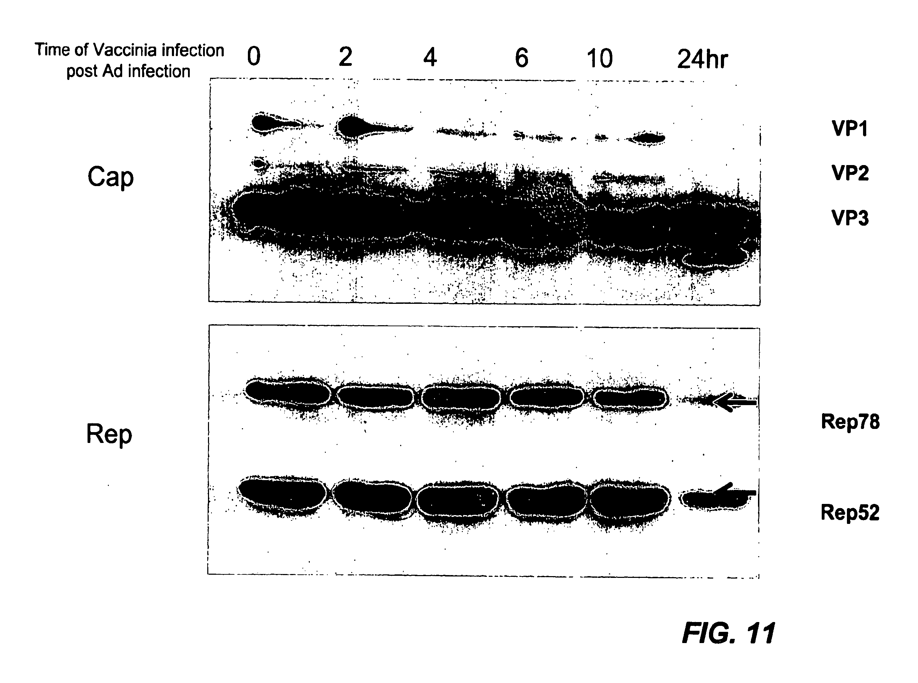

Hela cells were infected first with vAd-AAVITR-CMV-GFP at a multiplicity of infection (MOI) of 2, then with a mixture of equal amount of vvAAVrep78, vvAAVrep52, vvAAVvp1, vvAAVvp2, and vvAAVvp3 at an MOI of 1 at 12 hours post-vAd infection. An example of the rep and cap expression profile is shown figure 11. The cells were harvested 36 hours after wAAV virus infection. The recombinant AAV *(i.e.,* rAAV-CMV-GFP) was purified from the cell lysate using the ultracentrifugation with CsCl gradient as described by Current Protocols in Human Genetics, UNIT 12.9 Production of Recombinant Adeno-Associated Viral Vectors, by Choi et al.

In another experiment, Hela cells were infected first with vAd-AAVITR-CMV-GFP at a multiplicity of infection (MOI) of 2, then with a mixture of equal amount of vvAAVrep78, vvAAVrep52, vvAAVvp1, and vvAAVvp2 at an MOI of 1 at 12 hours post-vAd infection. The cells were harvested 36 hours after wAAV virus infection. The recombinant AAV (*i.e.,* rAAV-CMV-GFP) was purified from the cell lysate using the ultracentrifugation with CsCl gradient as described by Current Protocols in Human Genetics, UNIT 12.9 Production of Recombinant Adeno-Associated Viral Vectors, by Choi et al.

In another experiment, Hela cells were first infected with vAd-AAVITR-CMV-GFP, then infected with vvAAV-HI at 12 hours post-vAd infection at MOI of 2. The cells were harvested 36 hours after wAAV virus infection. The recombinant AAV was purified from the cell lysate as described in Current Protocols in Human Genetics, UNIT 12.9 Production of Recombinant Adeno-Associated Viral Vectors, by Choi et al.

### Example 7. Remove Vaccinia carries from recombinant vector preparations

Since Vaccinia virus is significant larger than other DNA viruses, it can be filtered from recombinant viral vector preparations by filtering the vectors through a 0.22um filter.

### Example 8. Production of recombinant AAV using vaccinia carrier viruses and cell line with integrated recombinant AA V genomes

Hela cells were stably transfected with pAAV-CMV-GFP. The resulting cell line, designated as Hela-AAV-CMV-GFP, was infected with either vvAAV-H1 or a combination of vvAAVrep78, vvAAVrep52, vvAAVvp1, vvAAVvp2, vvAAVvp3. The cell lysate containing recombinant AAV genomes were collected at 36 to 48 hours post infection. The recombinant AAV was purified from the cell lysate as described in Example 6.

### Example 9. Production of recombinant AAV using vaccinia carrier viruses and cell line with recombinant AAV genomes existing as episomes in the nucleus

AAVITR and CMV-GFP was cloned into the pRep4 vector (Invitrogen, Life Technologies Corp., Carlsbad, California), which contains EBNA and EBV latent origin and remains as episomes in the nucleus of the cells. The resulting plasmid, designated pRep4-AAV-CMV-GFP, was transfected into Hela cells. Hygromycin was added to the transfected cells to select cells with desired number of episomes. The selected cells were then infected with either vvAAV-H or a combination of vvAAVrep78, vvAAVrep52, vvAAVvp1, vvAAVvp2, vvAAVvp3. The cell lysate containing recombinant AAV genomes were collected at 36 to 48 hours post infection. The recombinant AAV was purified from the cell lysate as described in Example 6.

In this case, adenovirus helper function would improve the yield of recombinant AAV vectors, but the adenovirus helper function is not required for the generation of rAAV vectors.

### Example 10. Production of recombinant AAV using vaccinia carrier viruses in vivo in animal liver

vvAAVrep78, vvAVrep52, vvAAVvp1, vvAAVvp2, vvAAVvp3 and vAd-AAVITR-CMV-GFP are injected intravenously into immunodeficient Rag-1 mice. The liver of the mice are harvested 48 hours after virus infection. The recombinant AAV particles are purified from the liver tissue as described in Current Protocols in Human Genetics, UNIT 12.9: Production of Recombinant Adeno-Associated Viral Vectors, by Choi et al

Alternatively, vvAAV-H1 and vAd-AAVITR-CMV-GFP are injected intravenously intro immunodeficient Rag-1 mice. The liver of the mice are harvested 48 hours after virus infection. The recombinant AAV particles are purified from the liver tissue as described in Example 6.

The *in vivo* production of recombinant AAV requires no tissue culture reagent and equipment, and can easily scale up by using larger animals.

### Example 11. A model for continuous rAAV production

A cell line that is non-permissive for AAV infection, such as human megakaryocytic leukaemia cells (MB-02), is used for rAAV production. Briefly, MB-02 cells are infected with vvAAV-H1 and vAd-AAVITR-CMV-GFP in a suspension culture. The cells supernatant is harvested continuously. Fresh cells are added periodically. The recombinant AAV particles are purified from the harvested supernatant.

The advantage of this system is that the recombinant AAV particles released from the cells cannot re-enter the host cells and are released into the supernatant. This approach would greatly improve the yield of recombinant AAV production.

### Example 12. Use of VSV vector for AA V vector production

The vaccinia carrier vectors described in Examples 1-10 can be replaced by VSV carrier vectors VSV-rep78, VSV-Rep-52, VSV-VP1, VSV-VP2 and VSV-Vp3. Since VSV is a cytoplasmic RNA virus, it would prevent recombinantion between the recombination of rAAV genome and AAV functional genome sequences, which eliminate replication competent AAV particles formation. VSV carrier viruses can be generated as described by Miller et al., Protein Expr Purif. 2004, 33:92-103.

### Example 13, Vaccinia carrier vector for adenovirus vector production

In this example, vaccinia carrier virus is used to express some necessary genes for adenovirus production. Vaccinia carrier vector carrying adenovirus E1a, E1b and E4 genes will be generated as described in Example 1. Hela cells will be co-infected with recombinant adenovirus and vv-E1aE1bE4. The helper function provided by the vaccinia carrier virus would allow replication of the rAd in the Hela cells. The Hela cells will then be lysed and the recombinant adenovirus purified as described in Example 5. This system would allow quick production of recombinant adenovirus vector without the contamination of replication-competent adenovirus. The sequence for the trans cassette expressing human Ad5 E1a gene is shown in **SEQ ID NO:9.** The sequence for the trans cassette expressing human Ad5 E1b 55k gene is shown in **SEQ ID NO:10.** The sequence for the trans cassette expressing human Ad5 E2a gene is shown in **SEQ ID NO:11.** The sequence for the trans cassette expressing human Ad5 E4 gene is shown in **SEQ ID NO:12.**

### Example 14, Vaccinia carrier virus for replication-deficient HSV vector production

Because the HSV immediate early (IE) genes contribute to the toxicity of the virus, it is necessary to sequentially delete these cytotoxic genes from the viral vector genome. In addition, deletion of multiple genes from the virus increases the amount of foreign DNA that can be incorporated into the vector. Conventionally, in order to construct replication-defective genomic HSV type 1 (HSV-1) mutant vectors, it is necessary to first produce a cell line capable of synthesizing the IE gene product(s) required to complement the deletion virus. Our approach to elimination of the homologous recombination between the deletion virus and the HSV-1 gene is to express these essential early genes in vaccinia virus instead of a complementing cell line.

In this example, vaccinia carrier viruses are constructed to express genes necessary for hepesvirus production. In one embodiment, vaccinia carrier vectors carrying HSV-1 IE (ICP4 and ICP27) genes under the control vaccinia p7.5 promoter are generated using pRB21 plasmid, vRB12 virus and BSC cells as described above. The resulting vaccinia vectors are designated vvICP4-27. The recombinant herpes vector lacks the above genes but carrying CMV-GFP (vHerp-GFP) can be generated following the protocol described in Current Protocols in Human Genetics, UNIT 12.11 "Construction of Replication-Defective Herpes Simplex Virus Vectors" by William F. Goins et al. updated on January 14, 2010. The sequence for the trans cassette expressing human HSV-1 ICP4 gene is shown in **SEQ ID NO:13.** The sequence for the trans cassette expressing human HSV-1 ICP27 gene is shown in **SEQ ID NO:14.** To grow the HSV vectors in large scale, vvICP4-27 and vHerp-GFP were used to infect vero cells at a MOI of 2. After adsorption, decant the inoculum and add 100 to 125 ml complete MEM/10% FBS per bottle. Incubate at 37°C until all the cells have rounded up and are starting to detach from the surface. The HSV vector vHerp-GFP were then purified using the protocol described in the Current Protocols in Human Genetics, UNIT 12.11 "Construction of Replication-Defective Herpes Simplex Virus Vectors" by William F. Goins et al., updated on January 14, 2010.

Similarily, the vaccinia carrier virus can also be adapted for production of HSV amplicon vector. Cornel Fraefel, UNIT 12.12, Gene Delivery Using Helper Virus-Free HSV-1 Amplicon Vectors, Current Protocols in Human Genetics.

### Example 15. Production of recombinant parvovirus MVM vector using vaccinia carrier viruses and cell lines with integrated recombinant MVM genomes

NBK (human newborn kidney cell line) cells were stably transfected with pMVM-100a as described by Brandenburger et al. (Hum Gene Ther. 1999, 10:1229-1238). The positive clone (NBK-MVM-100a) with full recombinant MVM genome will be used for vector production. vvMVM-NS1, vvMVM-NS2, vvMVM-VP1 and vvMVM-VP2 will be constructed respectively to express NS1, NS2 , VP1 and VP2 proteins. NBK-MVM-100a will infected with the above-described four viruses at a MOI of 1 for each virus. The cell lysate containing recombinant MVM genomes were collected at 36 to 48 hours post infection. Recombinant parvovirus MVM vector will be isolated as described as described by Brandenburger and Velu,. J Gene Med. 2004,6 Suppl 1:S203-11.

### Example 16. RCA level in rAAV vectors preparation using a vaccinia carrier virus

### Human 293 cells were seeded at either 2.5x 10⁵ cells per T-25 flask or 5 x 10⁶

Human 293 cells were seeded at either 2.5x 10⁵ cells per T-25 flask or 5 x 10⁶ cells per 10-cm-diameter dish. The next day, the cultures were infected with adenovirus (MOI = 5) and infected with vector or wt AAV. After 72 h, either the cells were harvested and episomal DNA was isolated by the method of Hirt or the cells were scraped into the medium, subjected to three cycles of freeze-thaw in a dry ice-ethanol bath, and centrifuged to pellet the cell debris. The clarified crude lysate was incubated at 56°C for 1 h to inactivate the adenovirus, and 500 ml was added to a fresh plate of 293 cells with or without fresh adenovirus (MOI 5 5) for a second round of amplification. After an additional 72 h, the cells were scraped into the medium and pelleted, and total genomic DNA was prepared by standard techniques (31). The DNA was subjected to Southern analysis and hybridized with either a 32P-labeled BglII fragment from pAV2 comprising the entire AAV2 genome or a HindIII/SnaBI fragment of pAV2 containing the cap sequence.

The rAAV produced in example 6 showed that RCA were undetectable from 1x10¹¹ particles.

It should be noted that the above-described virus production can be done in suspension cells, since the carrier virus vector can infect host cells easily. In addition, since vaccinia virus has a diameter of about 0.23~0.4 µm, it can be conveniently removed from the recombinant viral vectors by filtering through a 0.22 µm column.

This invention thus has many advantages over current methods for manufacturing recombinant viral vectors. These advantages include: (1) the cytoplasm carrier viruses such as vaccinia virus has a large genome (about 300kb) that permits insertion of large DNA sequences without compromising the efficiency of recombinant viral vectors production; (2) the cytoplasmic carrier viruses will not meet the recombinant vector genome and therefore there will be no recombination events leading to the regeneration of replication competent viral particles; and (3) some cytoplasm carrier viruses, such as vaccinia virus, have a large size and can be easily removed from recombinant viral vector preparations.; and (4). The methods of the present invention are particularly suitable to large scale production of replication-deficient recombinant viral vectors since the time-consuming transfection based methods could be eliminated.

The herein described compositions and methods may be used to produce various recombinant viral vectors. The disclosure references particular means, materials and embodiments. Although the claims make reference to particular means, materials and embodiments, it is to be understood that the claims are not limited to these disclosed particulars, but extend instead to all equivalents.

## Claims

1. A method for eliminating or minimizing the formation of a replication-competent virus during the production of a replication-deficient recombinant virus vector having a recombinant virus genome with one or more defective viral genes, said method comprising:
infecting a host cell with a carrier virus having a carrier virus genome encoding one or more trans factors or variants thereof, wherein said carrier virus is a cytoplasmic virus that retains said carrier virus genome in the cytoplasm of said host cell, wherein said one or more trans factors or variants thereof compensate one or more functions of said one or more defective genes in said recombinant virus genome, and wherein said one or more trans factors or variants thereof comprise a structure protein of said replication-deficient recombinant virus;
incubating the infected host cell for a desired period of time; and
isolating said replication-deficient recombinant virus vector,
wherein said host cell contains said recombinant viral genome and retains said recombinant viral genome in a nucleus of said host cell.

2. The method of claim 1, wherein said carrier virus carries a carrier virus genome encoding two or more structure proteins of said replication-deficient recombinant virus and expresses said two or more structure proteins at a ratio suitable for the production of said replication-deficient recombinant virus.

3. The method of claim 1, wherein said cytoplasmic virus is a vaccinia virus or a vesicular stomatitis virus (VSV).

4. The method of claim 1, wherein said replication-deficient recombinant virus vector is a virus vector in the *Parvoviridae* family, a recombinant adenovirus vector, or a recombinant herpes virus vector.

5. A method for producing a replication-deficient recombinant virus vector having a recombinant virus genome with one or more defective viral genes, said method comprising:
infecting a host cell with a carrier virus having a carrier virus genome encoding one or more trans factors or variants thereof, wherein said carrier virus is a cytoplasmic virus that retains said carrier virus genome in the cytoplasm of said host cell and wherein said one or more trans factors or variants thereof compensate one or more functions of said one or more defective genes in said recombinant virus genome and wherein said one or more trans factors or variants thereof comprise a structure protein of said replication-deficient recombinant virus;
incubating the infected host cell for a desired period of time; and
isolating said replication-deficient recombinant virus vector,
wherein said host cell contains said recombinant viral genome and retains said recombinant viral genome in a nucleus of said host cell, and
wherein said replication-deficient recombinant virus vector is selected from the group consisting of recombinant virus vectors from the *Adenoviridae* family, the *Herpesviridae* family, the *Hepadnaviridae* family, and recombinant parvovirus vectors.

6. The method of claim 5, wherein said replication-deficient recombinant virus vector is a recombinant adenovirus vector, a recombinant herpes virus vector, or a recombinant parvovirus vector.

7. The method of claim 5, wherein said carrier virus is a vaccinia virus or vesicular stomatitis virus (VSV).

8. A method for producing a replication-deficient recombinant AAV vector having a recombinant virus genome that is defective in producing capsid proteins, said method comprising:
infecting a host cell with one or more carrier viruses, wherein said carrier viruses are cytoplasmic viruses that encode two or more AAV capsid proteins and express said two or more capsid proteins in the cytoplasm of said host cell in a ratio suitable for the production of said replication-deficient AAV vector;
incubating the infected host cell for a desired period of time; and
isolating said replication-deficient recombinant virus vector,
wherein said host cell contains said recombinant AAV genome and retains said recombinant AAV genome in a nucleus of said host cell.

9. The method of claim 8, wherein said two or more AAV capsid proteins are expressed under the control of vaccinia virus p7.5 promoter.

10. The method of claim 8, wherein said two or more AAV capsid proteins comprise VP2 and VP3 proteins expressed from a first vaccinia virus and VP1 expressed from a second vaccinia virus.

11. The method of claim 8, wherein the infecting step comprises infecting said host cell with a single vaccinia virus that expresses AAV VP1, VP2 and VP3 proteins at a ratio suitable for the production of said replication-deficient AAV vector, optionally wherein said single vaccinia virus contains a first expression cassette having an AAV vp1 gene under the control of a first vaccinia virus p7.5 promoter and a second expression cassette having an AAV vp2 gene under the control of a second vaccinia virus p7.5 promoter.

12. A carrier virus, comprising:
a capsid;
a carrier virus genome comprising:
a nucleic acid sequence from a cytoplasmic virus; and
a nucleotide sequence that encodes two or more AAV structure proteins or
variants thereof;
wherein said capsid and said nucleic acid sequence from a cytoplasmic virus allow said carrier virus genome to retain in the cytoplasm after entering a host cell, and wherein said carrier virus genome expresses said two or more AAV structure proteins or variants thereof in the cytoplasm at a ratio suitable for the production of a recombinant AAV vector that is defective in said two or more AAV structure proteins.

13. The carrier virus of claim 12, wherein said carrier virus is a vaccinia carrier virus or vesicular stomatitis virus (VSV), and wherein said virus genome comprises an expression cassette having an AAV vp1 gene under the control of a vaccinia virus p7.5 promoter when the carrier virus is a vaccinia virus.

14. The carrier virus of claim 12, wherein said carrier virus genome further comprises:
a nucleotide sequence that encodes one or more AAV non-structure proteins or variants thereof.

15. The carrier virus of claim 13, comprising a first expression cassette having an AAV vp1 gene under the control of a first vaccinia virus p7.5 promoter and a second expression cassette having an AAV vp2 gene under the control of a second vaccinia virus p7.5 promoter.

## Patentansprüche

1. Verfahren zur Eliminierung oder Minimierung der Bildung eines replikationskompetenten Virus während der Herstellung eines replikationsdefizienten rekombinanten Virusvektors, der ein rekombinantes Virusgenom mit einem oder mehreren defekten viralen Genen aufweist, wobei das genannte Verfahren Folgendes umfasst:
Infizieren einer Wirtszelle mit einem Trägervirus, der ein Trägervirusgenom aufweist, das einen oder mehrere trans-Faktoren oder Varianten davon kodiert, worin der genannte Trägervirus ein zytoplasmatischer Virus ist, der das genannte Trägervirusgenom im Zytoplasma der genannten Wirtszelle hält, worin der genannte eine oder die genannten mehreren trans-Faktoren oder Varianten davon eine oder mehrere Funktionen des genannten einen defekten Gens oder der genannten mehreren defekten Gene in dem genannten rekombinanten Virusgenom kompensieren und worin der genannte eine oder die genannten mehreren trans-Faktoren oder Varianten davon ein Strukturprotein des genannten replikationsdefizienten rekombinanten Virus umfassen;
Inkubieren der infizierten Wirtszelle für eine gewünschte Zeitdauer; und
Isolieren des genannten replikationsdefizienten rekombinanten Virusvektors,
worin die genannte Wirtszelle das genannte rekombinante virale Genom enthält und das genannte rekombinante virale Genom in einem Kern der genannten Wirtszelle hält.

2. Verfahren nach Anspruch 1, worin der genannte Trägervirus ein Trägervirusgenom enthält, das zwei oder mehr Strukturproteine des genannten replikationsdefizienten rekombinanten Virus kodiert, und die genannten zwei oder mehr Strukturproteine in einem Verhältnis exprimiert, das für die Herstellung des genannten replikationsdefizienten rekombinanten Virus geeignet ist.

3. Verfahren nach Anspruch 1, worin der genannte zytoplasmatische Virus ein Vaccinia-Virus oder ein Virus der vesikulären Stomatitis (VSV) ist.

4. Verfahren nach Anspruch 1, worin der genannte replikationsdefiziente rekombinante Virusvektor ein Virusvektor der *Parvoviridae*-Familie*,* ein rekombinanter Adenovirus-Vektor oder ein rekombinanter Herpesvirus-Vektor ist.

5. Verfahren zur Herstellung eines replikationsdefizienten rekombinanten Virusvektors, der ein rekombinantes Virusgenom mit einem oder mehreren defekten viralen Genen aufweist, wobei das genannte Verfahren Folgendes umfasst:
Infizieren einer Wirtszelle mit einem Trägervirus, der ein Trägervirusgenom aufweist, das einen oder mehrere trans-Faktoren oder Varianten davon kodiert, worin der genannte Trägervirus ein zytoplasmatischer Virus ist, der das genannte Trägervirusgenom im Zytoplasma der genannten Wirtszelle hält, und worin der genannte eine oder die genannten mehreren trans-Faktoren oder Varianten davon eine oder mehrere Funktionen des genannten einen defekten Gens oder der genannten mehreren defekten Gene in dem genannten rekombinanten Virusgenom kompensieren und worin der genannte eine oder die genannten mehreren trans-Faktoren oder Varianten davon ein Strukturprotein des genannten replikationsdefizienten rekombinanten Virus umfassen;
Inkubieren der infizierten Wirtszelle für eine gewünschte Zeitdauer; und
Isolieren des genannten replikationsdefizienten rekombinanten Virusvektors,
worin die genannte Wirtszelle das genannte rekombinante virale Genom enthält und das genannte rekombinante virale Genom in einem Kern der genannten Wirtszelle hält, und
worin der genannte replikationsdefiziente rekombinante Virusvektor aus der Gruppe ausgewählt ist, die aus rekombinanten Virusvektoren der *Adenoviridae*-Familie, der *Herpesviridae*-Familie, der *Hepadnaviridae*-Familie und rekombinanten Parvovirus-Vektoren besteht.

6. Verfahren nach Anspruch 5, worin der genannte replikationsdefiziente rekombinante Virusvektor ein rekombinanter Adenovirus-Vektor, ein rekombinanter Herpesvirus-Vektor oder ein rekombinanter Parvovirus-Vektor ist.

7. Verfahren nach Anspruch 5, worin der genannte Trägervirus ein Vaccinia-Virus oder ein Virus der vesikulären Stomatitis (VSV) ist.

8. Verfahren zur Herstellung eines replikationsdefizienten rekombinanten AAV-Vektors, der ein rekombinantes Virusgenom aufweist, das für die Herstellung von Capsid-Proteinen defekt ist, wobei das genannte Verfahren Folgendes umfasst:
Infizieren einer Wirtszelle mit einem oder mehreren Trägerviren, worin die genannten Trägerviren zytoplasmatische Viren sind, die zwei oder mehr AAV-Capsid-Proteine kodieren und die genannten zwei oder mehr Capsid-Proteine im Zytoplasma der genannten Wirtszelle in einem Verhältnis exprimieren, das für die Herstellung des genannten replikationsdefizienten AAV-Vektors geeignet ist;
Inkubieren der infizierten Wirtszelle für eine gewünschte Zeitdauer; und
Isolieren des genannten replikationsdefizienten rekombinanten Virusvektors,
worin die genannte Wirtszelle das genannte rekombinante AAV-Genom enthält und das genannte rekombinante AAV-Genom in einem Kern der genannten Wirtszelle hält.

9. Verfahren nach Anspruch 8, worin die genannten zwei oder mehr AAV-Capsid-Proteine unter der Kontrolle des Vaccinia-Virus-p7.5-Promotors exprimiert werden.

10. Verfahren nach Anspruch 8, worin die genannten zwei oder mehr AAV-Capsid-Proteine VP2- und VP3-Proteine, die von einem ersten Vaccinia-Virus exprimiert werden, und VP1 umfassen, das von einem zweiten Vaccinia-Virus exprimiert wird.

11. Verfahren nach Anspruch 8, worin der Infizierungsschritt das Infizieren der genannten Wirtszelle mit einem einzelnen Vaccinia-Virus umfasst, der AAV-VP1-, - VP2- und -VP3-Proteine in einem Verhältnis exprimiert, das für die Herstellung des genannten replikationsdefizienten AAV-Vektors geeignet ist, worin optional der genannte einzelne Vaccinia-Virus eine erste Expressionskassette, die ein AAV-vp1-Gen unter der Kontrolle eines ersten Vaccinia-Virus-p7.5-Promotors aufweist, und eine zweite Expressionskassette, die ein AAV-vp2-Gen unter der Kontrolle eines zweiten Vaccinia-Virus-p7.5-Promotors aufweist, enthält.

12. Trägervirus, der Folgendes umfasst:
ein Capsid;
ein Trägervirusgenom, das Folgendes umfasst:
eine Nukleinsäuresequenz aus einem zytoplasmatischen Virus; und
eine Nukleotidsequenz, die zwei oder mehr AAV-Strukturproteine oder
Varianten davon kodiert;
worin das genannte Capsid und die genannte Nucleinsäuresequenz aus einem zytoplasmatischen Virus dem genannten Trägervirusgenom erlauben, nach Eintritt in eine Wirtszelle im Zytoplasma zu verbleiben, und worin das genannte Trägervirusgenom die genannten zwei oder mehr AAV-Strukturproteine oder Varianten davon im Zytoplasma in einem Verhältnis exprimiert, das für die Herstellung eines rekombinanten AAV-Vektors geeignet ist, der in den genannten zwei oder mehr AAV-Strukturproteinen defekt ist.

13. Trägervirus nach Anspruch 12, worin der genannte Trägervirus ein Vaccinia-Trägervirus oder ein Virus der vesikulären Stomatitis (VSV) ist und worin das genannte Virusgenom eine Expressionskassette, die ein AAV-vp1-Gen unter der Kontrolle eines Vaccinia-Virus-p7.5-Promotors aufweist, umfasst, wenn der Trägervirus ein Vaccinia-Virus ist.

14. Trägervirus nach Anspruch 12, worin das genannte Trägervirusgenom weiter Folgendes umfasst:
eine Nukleotidsequenz, die ein oder mehrere AAV-Nichtstrukturproteine oder Varianten davon kodiert.

15. Trägervirus nach Anspruch 13, der eine erste Expressionskassette, die ein AAV-vp1-Gen unter der Kontrolle eines ersten Vaccinia-Virus-p7.5-Promotors aufweist, und eine zweite Expressionskassette, die ein AAV-vp2-Gen unter der Kontrolle eines zweiten Vaccinia-Virus-p7.5-Promotors aufweist, umfasst.

## Revendications

1. Méthode d'élimination ou de minimisation de la formation d'un virus compétent pour la réplication lors de la production d'un vecteur viral recombinant déficient pour la réplication ayant un génome viral recombinant qui comporte un ou plusieurs gènes viraux défectueux, ladite méthode comprenant :
l'infection d'une cellule hôte avec un virus porteur ayant un génome de virus porteur codant pour un ou plusieurs facteurs agissant en trans ou variants de ceux-ci, ledit virus porteur étant un virus cytoplasmique qui conserve ledit génome de virus porteur dans le cytoplasme de ladite cellule hôte, le(s)dit(s) un ou plusieurs facteurs agissant en trans ou variants de ceux-ci compensant une ou plusieurs fonctions du(des)dit(s) un ou plusieurs gènes défectueux dans ledit génome viral recombinant et le(s)dit(s) un ou plusieurs facteurs agissant en trans ou variants de ceux-ci comprenant une protéine structurale dudit virus recombinant déficient pour la réplication ;
l'incubation de la cellule hôte infectée pendant une période de temps désirée ; et
l'isolation dudit vecteur viral recombinant déficient pour la réplication,
dans laquelle ladite cellule hôte contient ledit génome viral recombinant et conserve ledit génome viral recombinant dans un noyau de ladite cellule hôte.

2. Méthode de revendication 1, dans laquelle ledit virus porteur porte un génome de virus porteur codant pour deux ou plusieurs protéines structurales dudit virus recombinant déficient pour la réplication et exprime lesdites deux ou plusieurs protéines structurales dans un rapport approprié pour la production dudit virus recombinant déficient pour la réplication.

3. Méthode de revendication 1, dans laquelle ledit virus cytoplasmique est un virus de la vaccine ou un virus de la stomatite vésiculeuse (VSV).

4. Méthode de revendication 1, dans laquelle ledit vecteur viral recombinant déficient pour la réplication est un vecteur viral de la famille des *Parvoviridae,* un vecteur recombinant dérivé d'un adénovirus ou un vecteur recombinant dérivé d'un herpèsvirus.

5. Méthode de production d'un vecteur viral recombinant déficient pour la réplication ayant un génome viral recombinant qui comporte un ou plusieurs gènes viraux défectueux, ladite méthode comprenant :
l'infection d'une cellule hôte avec un virus porteur ayant un génome de virus porteur codant pour un ou plusieurs facteurs agissant en trans ou variants de ceux-ci, ledit virus porteur étant un virus cytoplasmique qui conserve ledit génome de virus porteur dans le cytoplasme de ladite cellule hôte, le(s)dit(s) un ou plusieurs facteurs agissant en trans ou variants de ceux-ci compensant une ou plusieurs fonctions du(des)dit(s) un ou plusieurs gènes défectueux dans ledit génome viral recombinant et le(s)dit(s) un ou plusieurs facteurs agissant en trans ou variants de ceux-ci comprenant une protéine structurale dudit virus recombinant déficient pour la réplication ;
l'incubation de la cellule hôte infectée pendant une période de temps désirée ; et
l'isolation dudit vecteur viral recombinant déficient pour la réplication,
dans laquelle ladite cellule hôte contient ledit génome viral recombinant et conserve ledit génome viral recombinant dans un noyau de ladite cellule hôte et
dans laquelle ledit vecteur viral recombinant déficient pour la réplication est sélectionné dans le groupe consistant en des vecteurs viraux de la famille des *Adenoviridae,* de la famille des *Herpesviridae* ou de la famille de *Hepadnaviridae* et des vecteurs recombinants dérivés d'un parvovirus

6. Méthode de revendication 5, dans laquelle ledit vecteur viral recombinant déficient pour la réplication est un vecteur recombinant dérivé d'un adénovirus, un vecteur recombinant dérivé d'un herpèsvirus ou un vecteur recombinant dérivé d'un parvovirus.

7. Méthode de revendication 5, dans laquelle ledit virus porteur est un virus de la vaccine ou un virus de la stomatite vésiculeuse (VSV).

8. Méthode de production d'un vecteur recombinant déficient pour la réplication dérivé d'un virus associé à un adénovirus (AAV) ayant un génome viral recombinant qui est défectueux en produisant des protéines de la capside, ladite méthode comprenant :
l'infection d'une cellule hôte avec un ou plusieurs virus porteurs, lesdits virus porteurs étant des virus cytoplasmiques qui codent pour deux ou plusieurs protéines de la capside de l'AAV et expriment lesdites deux ou plusieurs protéines de la capside dans le cytoplasme de ladite cellule hôte dans un rapport approprié pour la production dudit vecteur déficient pour la réplication dérivé d'un AAV ;
l'incubation de la cellule hôte infectée pendant une période de temps désirée ; et
l'isolation dudit vecteur viral recombinant déficient pour la réplication,
dans laquelle ladite cellule hôte contient ledit génome de l'AAV recombinant et conserve ledit génome de l'AAV recombinant dans un noyau de ladite cellule hôte.

9. Méthode de revendication 8, dans laquelle lesdites deux ou plusieurs protéines de la capside de l'AAV sont exprimées sous le contrôle du promoteur p7.5 du virus de la vaccine.

10. Méthode de revendication 8, dans laquelle lesdites deux ou plusieurs protéines de la capside de l'AAV comprennent les protéines VP2 et VP3 exprimées à partir d'un premier virus de la vaccine et la protéine VP1 exprimée à partir d'un deuxième virus de la vaccine.

11. Méthode de revendication 8, dans laquelle l'étape d'infection comprend l'infection de ladite cellule hôte avec un virus de la vaccine unique qui exprime les protéines VP1, VP2 et VP3 de l'AAV dans un rapport approprié pour la production dudit vecteur déficient pour la réplication dérivé d'un AAV, ledit virus de la vaccine unique contenant en option une première cassette d'expression ayant un gène vp1 de l'AAV sous le contrôle du promoteur p7.5 d'un premier virus de la vaccine et une deuxième cassette d'expression ayant un gène vp2 de l'AAV sous contrôle du promoteur p7.5 d'un deuxième virus de la vaccine.

12. Virus porteur comprenant :
une capside ;
un génome de virus porteur comprenant :
une séquence d'acide nucléique d'un virus cytoplasmique ; et
une séquence nucléotidique qui code pour deux ou plusieurs protéines structurales de l'AAV ou variants de celles-ci ;
dans lequel ladite capside et ladite séquence d'acide nucléique d'un virus cytoplasmique permettent au dit génome de virus porteur de rester dans le cytoplasme après être entré dans une cellule hôte et dans lequel ledit génome de virus porteur exprime lesdites deux ou plusieurs protéines structurales de l'AAV ou variants de celles-ci dans le cytoplasme dans un rapport approprié pour la production d'un vecteur recombinant dérivé de l'AAV qui est déficient en lesdites deux ou plusieurs protéines structurales de l'AAV.

13. Virus porteur de la revendication 12, dans lequel ledit virus porteur est un virus porteur de la vaccine ou un virus de la stomatite vésiculeuse (VSV) et dans lequel ledit génome du virus comprend une cassette d'expression ayant un gène vp1 de l'AAV sous le contrôle du promoteur p7.5 du virus de la vaccine quand le virus porteur est un virus de la vaccine.

14. Virus porteur de la revendication 12, dans lequel ledit génome du virus porteur comprend également :
une séquence nucléotidique qui code pour deux ou plusieurs protéines non structurales de l'AVV ou variants de celles-ci.

15. Virus porteur de la revendication 13, qui comprend une première cassette d'expression ayant un gène vp1 de l'AAV sous le contrôle du promoteur p7.5 d'un premier virus de la vaccine et une deuxième cassette d'expression ayant un gène vp2 de l'AAV sous contrôle du promoteur p7.5 d'un deuxième virus de la vaccine.


### SEQUENCE LISTING

<110> Xiao, weidong
<120> Methods and compositions for the production of recombinant virus vectors
<130> 191386
<160> 14
<170> PatentIn version 3.5
<210> 1
   <211> 39
   <212> DNA
   <213> vaccinia virus
<400> 1
   aaaaattgaa attttatttt ttttttttgg aatataaat 39
<210> 2
   <211> 1963
   <212> DNA
   <213> artificial
<220>
   <223> expression cassette
<400> 2
   aaaaattgaa attttatttt ttttttttgg aatataaata aggaattcct gcaggctagc 60
   cccgggaagc ttccatgccg gggttttacg agattgtgat taaggtcccc agcgaccttg 120
   acgagcatct gcccggcatt tctgacagct ttgtgaactg ggtggccgag aaggaatggg 180
   agttgccgcc agattctgac atggatctga atctgattga gcaggcaccc ctgaccgtgg 240
   ccgagaagct gcagcgcgac tttctgacgg aatggcgccg tgtgagtaag gccccggagg 300
   cccttttctt tgtgcaattt gagaagggag agagctactt ccacatgcac gtgctcgtgg 360
   aaaccaccgg ggtgaaatcc atggttttgg gacgtttcct gagtcagatt cgcgaaaaac 420
   tgattcagag aatttaccgc gggatcgagc cgactttgcc aaactggttc gcggtcacaa 480
   agaccagaaa tggcgccgga ggcgggaaca aggtggtgga tgagtgctac atccccaatt 540
   acttgctccc caaaacccag cctgagctcc agtgggcgtg gactaatatg gaacagtatt 600
   taagcgcctg tttgaatctc acggagcgta aacggttggt ggcgcagcat ctgacgcacg 660
   tgtcgcagac gcaggagcag aacaaagaga atcagaatcc caattctgat gcgccggtga 720
   tcagatcaaa aacttcagcc aggtacatgg agctggtcgg gtggctcgtg gacaagggga 780
   ttacctcgga gaagcagtgg atccaggagg accaggcctc atacatctcc ttcaatgcgg 840
   cctccaactc gcggtcccaa atcaaggctg ccttggacaa tgcgggaaag attatgagcc 900
   tgactaaaac cgcccccgac tacctggtgg gccagcagcc cgtggaggac atttccagca 960
   atcggattta taaaattttg gaactaaacg ggtacgatcc ccaatatgcg gcttccgtct 1020
   ttctgggatg ggccacgaaa aagttcggca agaggaacac catctggctg tttgggcctg 1080
   caactaccgg gaagaccaac atcgcggagg ccatagccca cactgtgccc ttctacgggt 1140
   gcgtaaactg gaccaatgag aactttccct tcaacgactg tgtcgacaag atggtgatct   1200
   ggtgggagga ggggaagatg accgccaagg tcgtggagtc ggccaaagcc attctcggag   1260
   gaagcaaggt gcgcgtggac cagaaatgca agtcctcggc ccagatagac ccgactcccg   1320
   tgatcgtcac ctccaacacc aacatgtgcg ccgtgattga cgggaactca acgaccttcg   1380
   aacaccagca gccgttgcaa gaccggatgt tcaaatttga actcacccgc cgtctggatc   1440
   atgactttgg gaaggtcacc aagcaggaag tcaaagactt tttccggtgg gcaaaggatc   1500
   acgtggttga ggtggagcat gaattctacg tcaaaaaggg tggagccaag aaaagacccg   1560
   cccccagtga cgcagatata agtgagccca aacgggtgcg cgagtcagtt gcgcagccat   1620
   cgacgtcaga cgcggaagct tcgatcaact acgcagacag gtaccaaaac aaatgttctc   1680
   gtcacgtggg catgaatctg atgctgtttc cctgcagaca atgcgagaga atgaatcaga   1740
   attcaaatat ctgcttcact cacggacaga aagactgttt agagtgcttt cccgtgtcag   1800
   aatctcaacc cgtttctgtc gtcaaaaagg cgtatcagaa actgtgctac attcatcata   1860
   tcatgggaaa ggtgccagac gcttgcactg cctgcgatct ggtcaatgtg gatttggatg   1920
   actgcatctt tgaacaataa aggcctaaat aaataatttt tat                     1963
<210> 3
   <211> 2029
   <212> DNA
   <213> artificial
<220>
   <223> expression cassette
<400> 3
   aaaaattgaa attttatttt ttttttttgg aatataaata aggaattcct gcaggctagc   60
   cccgggaagc ttccatgccg gggttttacg agattgtgat taaggtcccc agcgaccttg   120
   acgagcatct gcccggcatt tctgacagct ttgtgaactg ggtggccgag aaggaatggg   180
   agttgccgcc agattctgac atggatctga atctgattga gcaggcaccc ctgaccgtgg   240
   ccgagaagct gcagcgcgac tttctgacgg aatggcgccg tgtgagtaag gccccggagg   300
   cccttttctt tgtgcaattt gagaagggag agagctactt ccacatgcac gtgctcgtgg   360
   aaaccaccgg ggtgaaatcc atggttttgg gacgtttcct gagtcagatt cgcgaaaaac   420
   tgattcagag aatttaccgc gggatcgagc cgactttgcc aaactggttc gcggtcacaa   480
   agaccagaaa tggcgccgga ggcgggaaca aggtggtgga tgagtgctac atccccaatt   540
   acttgctccc caaaacccag cctgagctcc agtgggcgtg gactaatatg gaacagtatt   600
   taagcgcctg tttgaatctc acggagcgta aacggttggt ggcgcagcat ctgacgcacg   660
   tgtcgcagac gcaggagcag aacaaagaga atcagaatcc caattctgat gcgccggtga   720
   tcagatcaaa aacttcagcc aggtacatgg agctggtcgg gtggctcgtg gacaagggga   780
   ttacctcgga gaagcagtgg atccaggagg accaggcctc atacatctcc ttcaatgcgg   840
   cctccaactc gcggtcccaa atcaaggctg ccttggacaa tgcgggaaag attatgagcc   900
   tgactaaaac cgcccccgac tacctggtgg gccagcagcc cgtggaggac atttccagca 960
   atcggattta taaaattttg gaactaaacg ggtacgatcc ccaatatgcg gcttccgtct 1020
   ttctgggatg ggccacgaaa aagttcggca agaggaacac catctggctg tttgggcctg 1080
   caactaccgg gaagaccaac atcgcggagg ccatagccca cactgtgccc ttctacgggt 1140
   gcgtaaactg gaccaatgag aactttccct tcaacgactg tgtcgacaag atggtgatct 1200
   ggtgggagga ggggaagatg accgccaagg tcgtggagtc ggccaaagcc attctcggag 1260
   gaagcaaggt gcgcgtggac cagaaatgca agtcctcggc ccagatagac ccgactcccg 1320
   tgatcgtcac ctccaacacc aacatgtgcg ccgtgattga cgggaactca acgaccttcg 1380
   aacaccagca gccgttgcaa gaccggatgt tcaaatttga actcacccgc cgtctggatc 1440
   atgactttgg gaaggtcacc aagcaggaag tcaaagactt tttccggtgg gcaaaggatc 1500
   acgtggttga ggtggagcat gaattctacg tcaaaaaggg tggagccaag aaaagacccg 1560
   cccccagtga cgcagatata agtgagccca aacgggtgcg cgagtcagtt gcgcagccat 1620
   cgacgtcaga cgcggaagct tcgatcaact acgcagacag gtaccaaaac aaatgttctc 1680
   gtcacgtggg catgaatctg atgctgtttc cctgcagaca atgcgagaga atgaatcaga 1740
   attcaaatat ctgcttcact cacggacaga aagactgttt agagtgcttt cccgtgtcag 1800
   aatctcaacc cgtttctgtc gtcaaaaagg cgtatcagaa actgtgctac attcatcata 1860
   tcatgggaaa ggtgccagac gcttgcactg cctgcgatct ggtcaatgtg gatttggatg 1920
   actgcatctt tgaacaataa atgatttaaa tcaggtatgg ctgccgatgg ttatcttcca 1980
   gattggctcg aggacactct ctctgaaggc ctaaataaat aatttttat 2029
<210> 4
   <211> 1291
   <212> DNA
   <213> artificial
<220>
   <223> expression cassette
<400> 4
   aaaaattgaa attttatttt ttttttttgg aatataaata aggaattcct gcaggctagc      60
   cccgggaagc ttccatggag ctggtcgggt ggctcgtgga caaggggatt acctcggaga     120
   agcagtggat ccaggaggac caggcctcat acatctcctt caatgcggcc tccaactcgc     180
   ggtcccaaat caaggctgcc ttggacaatg cgggaaagat tatgagcctg actaaaaccg     240
   cccccgacta cctggtgggc cagcagcccg tggaggacat ttccagcaat cggatttata     300
   aaattttgga actaaacggg tacgatcccc aatatgcggc ttccgtcttt ctgggatggg     360
   ccacgaaaaa gttcggcaag aggaacacca tctggctgtt tgggcctgca actaccggga     420
   agaccaacat cgcggaggcc atagcccaca ctgtgccctt ctacgggtgc gtaaactgga     480
   ccaatgagaa ctttcccttc aacgactgtg tcgacaagat ggtgatctgg tgggaggagg     540
   ggaagatgac cgccaaggtc gtggagtcgg ccaaagccat tctcggagga agcaaggtgc     600
   gcgtggacca gaaatgcaag tcctcggccc agatagaccc gactcccgtg atcgtcacct     660
   ccaacaccaa catgtgcgcc gtgattgacg ggaactcaac gaccttcgaa caccagcagc     720
   cgttgcaaga ccggatgttc aaatttgaac tcacccgccg tctggatcat gactttggga     780
   aggtcaccaa gcaggaagtc aaagactttt tccggtgggc aaaggatcac gtggttgagg     840
   tggagcatga attctacgtc aaaaagggtg gagccaagaa aagacccgcc cccagtgacg     900
   cagatataag tgagcccaaa cgggtgcgcg agtcagttgc gcagccatcg acgtcagacg     960
   cggaagcttc gatcaactac gcagacaggt accaaaacaa atgttctcgt cacgtgggca    1020
   tgaatctgat gctgtttccc tgcagacaat gcgagagaat gaatcagaat tcaaatatct    1080
   gcttcactca cggacagaaa gactgtttag agtgctttcc cgtgtcagaa tctcaacccg    1140
   tttctgtcgt caaaaaggcg tatcagaaac tgtgctacat tcatcatatc atgggaaagg    1200
   tgccagacgc ttgcactgcc tgcgatctgg tcaatgtgga tttggatgac tgcatctttg    1260
   aacaataaag gcctaaataa ataattttta t                                   1291
<210> 5
   <211> 1357
   <212> DNA
   <213> artificial
<220>
   <223> expression cassette
<400> 5
   aaaaattgaa atttattttt ttttttttgg aatataaata aggaattcct gcaggctagc        60
   cccgggaagc ttccatggag ctggtcgggt ggctcgtgga caaggggatt acctcggaga      120
   agcagtggat ccaggaggac caggcctcat acatctcctt caatgcggcc tccaactcgc      180
   ggtcccaaat caaggctgcc ttggacaatg cgggaaagat tatgagcctg actaaaaccg      240
   cccccgacta cctggtgggc cagcagcccg tggaggacat ttccagcaat cggatttata      300
   aaattttgga actaaacggg tacgatcccc aatatgcggc ttccgtcttt ctgggatggg      360
   ccacgaaaaa gttcggcaag aggaacacca tctggctgtt tgggcctgca actaccggga      420
   agaccaacat cgcggaggcc atagcccaca ctgtgccctt ctacgggtgc gtaaactgga      480
   ccaatgagaa ctttcccttc aacgactgtg tcgacaagat ggtgatctgg tgggaggagg      540
   ggaagatgac cgccaaggtc gtggagtcgg ccaaagccat tctcggagga agcaaggtgc      600
   gcgtggacca gaaatgcaag tcctcggccc agatagaccc gactcccgtg atcgtcacct      660
   ccaacaccaa catgtgcgcc gtgattgacg ggaactcaac gaccttcgaa caccagcagc      720
   cgttgcaaga ccggatgttc aaatttgaac tcacccgccg tctggatcat gactttggga      780
   aggtcaccaa gcaggaagtc aaagactttt tccggtgggc aaaggatcac gtggttgagg      840
   tggagcatga attctacgtc aaaaagggtg gagccaagaa aagacccgcc cccagtgacg      900
   cagatataag tgagcccaaa cgggtgcgcg agtcagttgc gcagccatcg acgtcagacg      960
   cggaagcttc gatcaactac gcagacaggt accaaaacaa atgttctcgt cacgtgggca     1020
   tgaatctgat gctgtttccc tgcagacaat gcgagagaat gaatcagaat tcaaatatct     1080
   gcttcactca cggacagaaa gactgtttag agtgctttcc cgtgtcagaa tctcaacccg     1140
   tttctgtcgt caaaaaggcg tatcagaaac tgtgctacat tcatcatatc atgggaaagg     1200
   tgccagacgc ttgcactgcc tgcgatctgg tcaatgtgga tttggatgac tgcatctttg     1260
   aacaataaat gatttaaatc aggtatggct gccgatggtt atcttccaga ttggctcgag     1320
   gacactctct ctgaaggcct aaataaataa tttttat                               1357
<210> 6
   <211> 2291
   <212> DNA
   <213> artificial
<220>
   <223> expression cassette
<400> 6
   aaaaattgaa attttatttt ttttttttgg aatataaata aggaattcct gcaggctagc 60
   cccatggctg ccgatggtta tcttccagat tggctcgagg acactctctc tgaaggaata 120
   agacagtggt ggaagctcaa acctggccca ccaccaccaa agcccgcaga gcggcataag 180
   gacgacagca ggggtcttgt gcttcctggg tacaagtacc tcggaccctt caacggactc 240
   gacaagggag agccggtcaa cgaggcagac gccgcggccc tcgagcacga caaagcctac 300
   gaccggcagc tcgacagcgg agacaacccg tacctcaagt acaaccacgc cgacgcggag 360
   tttcaggagc gccttaaaga agatacgtct tttgggggca acctcggacg agcagtcttc 420
   caggcgaaaa agagggttct tgaacctctg ggcctggttg aggaacctgt taagacggct 480
   ccgggaaaaa agaggccggt agagcactct cctgtggagc cagactcctc ctcgggaacc 540
   ggaaaggcgg gccagcagcc tgcaagaaaa agattgaatt ttggtcagac tggagacgca 600
   gactcagtac ctgaccccca gcctctcgga cagccaccag cagccccctc tggtctggga 660
   actaatacga tggctacagg cagtggcgca ccaatggcag acaataacga gggcgccgac 720
   ggagtgggta attcctcggg aaattggcat tgcgattcca catggatggg cgacagagtc 780
   atcaccacca gcacccgaac ctgggccctg cccacctaca acaaccacct ctacaaacaa 840
   atttccagcc aatcaggagc ctcgaacgac aatcactact ttggctacag caccccttgg 900
   gggtattttg acttcaacag attccactgc cacttttcac cacgtgactg gcaaagactc 960
   atcaacaaca actggggatt ccgacccaag agactcaact tcaagctctt taacattcaa 1020
   gtcaaagagg tcacgcagaa tgacggtacg acgacgattg ccaataacct taccagcacg 1080
   gttcaggtgt ttactgactc ggagtaccag ctcccgtacg tcctcggctc ggcgcatcaa 1140
   ggatgcctcc cgccgttccc agcagacgtc ttcatggtgc cacagtatgg atacctcacc 1200
   ctgaacaacg ggagtcaggc agtaggacgc tcttcatttt actgcctgga gtactttcct 1260
   tctcagatgc tgcgtaccgg aaacaacttt accttcagct acacttttga ggacgttcct 1320
   ttccacagca gctacgctca cagccagagt ctggaccgtc tcatgaatcc tctcatcgac 1380
   cagtacctgt attacttgag cagaacaaac actccaagtg gaaccaccac gcagtcaagg    1440
   cttcagtttt ctcaggccgg agcgagtgac attcgggacc agtctaggaa ctggcttcct    1500
   ggaccctgtt accgccagca gcgagtatca aagacatctg cggataacaa caacagtgaa    1560
   tactcgtgga ctggagctac caagtaccac ctcaatggca gagactctct ggtgaatccg    1620
   ggcccggcca tggcaagcca caaggacgat gaagaaaagt tttttcctca gagcggggtt    1680
   ctcatctttg ggaagcaagg ctcagagaaa acaaatgtgg acattgaaaa ggtcatgatt    1740
   acagacgaag aggaaatcag gacaaccaat cccgtggcta cggagcagta tggttctgta    1800
   tctaccaacc tccagagagg caacagacaa gcagctaccg cagatgtcaa cacacaaggc    1860
   gttcttccag gcatggtctg gcaggacaga gatgtgtacc ttcaggggcc catctgggca    1920
   aagattccac acacggacgg acattttcac ccctctcccc tcatgggtgg attcggactt    1980
   aaacaccctc ctccacagat tctcatcaag aacaccccgg tacctgcgaa tccttcgacc    2040
   accttcagtg cggcaaagtt tgcttccttc atcacacagt actccacggg acaggtcagc    2100
   gtggagatcg agtgggagct gcagaaggaa aacagcaaac gctggaatcc cgaaattcag    2160
   tacacttcca actacaacaa gtctgttaat gtggacttta ctgtggacac taatggcgtg    2220
   tattcagagc ctcgccccat tggcaccaga tacctgactc gtaatctgta acctaaataa    2280
   ataattttta t                                                        2291
<210> 7
   <211> 1880
   <212> DNA
   <213> artificial
<220>
   <223> expression cassette
<400> 7
   aaaaattgaa attttatttt ttttttttgg aatataaata aggaattcct gcaggctagc 60
   cccacggctc cgggaaaaaa gaggccggta gagcactctc ctgtggagcc agactcctcc 120
   tcgggaaccg gaaaggcggg ccagcagcct gcaagaaaaa gattgaattt tggtcagact 180
   ggagacgcag actcagtacc tgacccccag cctctcggac agccaccagc agccccctct 240
   ggtctgggaa ctaatacgat ggctacaggc agtggcgcac caatggcaga caataacgag 300
   ggcgccgacg gagtgggtaa ttcctcggga aattggcatt gcgattccac atggatgggc 360
   gacagagtca tcaccaccag cacccgaacc tgggccctgc ccacctacaa caaccacctc 420
   tacaaacaaa tttccagcca atcaggagcc tcgaacgaca atcactactt tggctacagc 480
   accccttggg ggtattttga cttcaacaga ttccactgcc acttttcacc acgtgactgg 540
   caaagactca tcaacaacaa ctggggattc cgacccaaga gactcaactt caagctcttt 600
   aacattcaag tcaaagaggt cacgcagaat gacggtacga cgacgattgc caataacctt 660
   accagcacgg ttcaggtgtt tactgactcg gagtaccagc tcccgtacgt cctcggctcg 720
   gcgcatcaag gatgcctccc gccgttccca gcagacgtct tcatggtgcc acagtatgga 780
   tacctcaccc tgaacaacgg gagtcaggca gtaggacgct cttcatttta ctgcctggag 840
   tactttcctt ctcagatgct gcgtaccgga aacaacttta ccttcagcta cacttttgag 900
   gacgttcctt tccacagcag ctacgctcac agccagagtc tggaccgtct catgaatcct 960
   ctcatcgacc agtacctgta ttacttgagc agaacaaaca ctccaagtgg aaccaccacg 1020
   cagtcaaggc ttcagttttc tcaggccgga gcgagtgaca ttcgggacca gtctaggaac 1080
   tggcttcctg gaccctgtta ccgccagcag cgagtatcaa agacatctgc ggataacaac 1140
   aacagtgaat actcgtggac tggagctacc aagtaccacc tcaatggcag agactctctg 1200
   gtgaatccgg gcccggccat ggcaagccac aaggacgatg aagaaaagtt ttttcctcag 1260
   agcggggttc tcatctttgg gaagcaaggc tcagagaaaa caaatgtgga cattgaaaag 1320
   gtcatgatta cagacgaaga ggaaatcagg acaaccaatc ccgtggctac ggagcagtat 1380
   ggttctgtat ctaccaacct ccagagaggc aacagacaag cagctaccgc agatgtcaac 1440
   acacaaggcg ttcttccagg catggtctgg caggacagag atgtgtacct tcaggggccc 1500
   atctgggcaa agattccaca cacggacgga cattttcacc cctctcccct catgggtgga 1560
   ttcggactta aacaccctcc tccacagatt ctcatcaaga acaccccggt acctgcgaat 1620
   ccttcgacca ccttcagtgc ggcaaagttt gcttccttca tcacacagta ctccacggga 1680
   caggtcagcg tggagatcga gtgggagctg cagaaggaaa acagcaaacg ctggaatccc 1740
   gaaattcagt acacttccaa ctacaacaag tctgttaatg tggactttac tgtggacact 1800
   aatggcgtgt attcagagcc tcgccccatt ggcaccagat acctgactcg taatctgtaa 1860
   cctaaataaa taatttttat 1880
<210> 8
   <211> 1699
   <212> DNA
   <213> artificial
<220>
   <223> expression cassette
<400> 8
   aaaaattgaa attttatttt ttttttttgg aatataaata aggaattcct gcaggctagc      60
   cccgggaagc ttccatggct acaggcagtg gcgcaccaat ggcagacaat aacgagggcg     120
   ccgacggagt gggtaattcc tcgggaaatt ggcattgcga ttccacatgg atgggcgaca     180
   gagtcatcac caccagcacc cgaacctggg ccctgcccac ctacaacaac cacctctaca     240
   aacaaatttc cagccaatca ggagcctcga acgacaatca ctactttggc tacagcaccc     300
   cttgggggta ttttgacttc aacagattcc actgccactt ttcaccacgt gactggcaaa     360
   gactcatcaa caacaactgg ggattccgac ccaagagact caacttcaag ctctttaaca     420
   ttcaagtcaa agaggtcacg cagaatgacg gtacgacgac gattgccaat aaccttacca     480
   gcacggttca ggtgtttact gactcggagt accagctccc gtacgtcctc ggctcggcgc     540
   atcaaggatg cctcccgccg ttcccagcag acgtcttcat ggtgccacag tatggatacc     600
   tcaccctgaa caacgggagt caggcagtag gacgctcttc attttactgc ctggagtact      660
   ttccttctca gatgctgcgt accggaaaca actttacctt cagctacact tttgaggacg     720
   ttcctttcca cagcagctac gctcacagcc agagtctgga ccgtctcatg aatcctctca     780
   tcgaccagta cctgtattac ttgagcagaa caaacactcc aagtggaacc accacgcagt     840
   caaggcttca gttttctcag gccggagcga gtgacattcg ggaccagtct aggaactggc     900
   ttcctggacc ctgttaccgc cagcagcgag tatcaaagac atctgcggat aacaacaaca     960
   gtgaatactc gtggactgga gctaccaagt accacctcaa tggcagagac tctctggtga    1020
   atccgggccc ggccatggca agccacaagg acgatgaaga aaagtttttt cctcagagcg    1080
   gggttctcat ctttgggaag caaggctcag agaaaacaaa tgtggacatt gaaaaggtca    1140
   tgattacaga cgaagaggaa atcaggacaa ccaatcccgt ggctacggag cagtatggtt    1200
   ctgtatctac caacctccag agaggcaaca gacaagcagc taccgcagat gtcaacacac    1260
   aaggcgttct tccaggcatg gtctggcagg acagagatgt gtaccttcag gggcccatct    1320
   gggcaaagat tccacacacg gacggacatt ttcacccctc tcccctcatg ggtggattcg    1380
   gacttaaaca ccctcctcca cagattctca tcaagaacac cccggtacct gcgaatcctt    1440
   cgaccacctt cagtgcggca aagtttgctt ccttcatcac acagtactcc acgggacagg    1500
   tcagcgtgga gatcgagtgg gagctgcaga aggaaaacag caaacgctgg aatcccgaaa    1560
   ttcagtacac ttccaactac aacaagtctg ttaatgtgga ctttactgtg gacactaatg    1620
   gcgtgtattc agagcctcgc cccattggca ccagatacct gactcgtaat ctgtaaaggc    1680
   ctaaataaat aatttttat                                                1699
<210> 9
   <211> 1083
   <212> DNA
   <213> artificial
<220>
   <223> expression cassette
<400> 9
   aaaaattgaa attttatttt ttttttttgg aatataaata aggaattcct gcaggctagc      60
   cccgggaagc ttccatgaga catattatct gccacggagg tgttattacc gaagaaatgg    120
   ccgccagtct tttggaccag ctgatcgaag aggtactggc tgataatctt ccacctccta    180
   gccattttga accacctacc cttcacgaac tgtatgattt agacgtgacg gcccccgaag    240
   atcccaacga ggaggcggtt tcgcagattt ttcccgactc tgtaatgttg gcggtgcagg    300
   aagggattga cttactcact tttccgccgg cgcccggttc tccggagccg cctcaccttt    360
   cccggcagcc cgagcagccg gagcagagag ccttgggtcc ggtttctatg ccaaaccttg    420
   taccggaggt gatcgatctt acctgccacg aggctggctt tccacccagt gacgacgagg    480
   atgaagaggg tgaggagttt gtgttagatt atgtggagca ccccgggcac ggttgcaggt    540
   cttgtcatta tcaccggagg aatacggggg acccagatat tatgtgttcg ctttgctata    600
   tgaggacctg tggcatgttt gtctacagta agtgaaaatt atgggcagtg ggtgatagag    660
   tggtgggttt ggtgtggtaa tttttttttt aatttttaca gttttgtggt ttaaagaatt    720
   ttgtattgtg atttttttaa aaggtcctgt gtctgaacct gagcctgagc ccgagccaga    780
   accggagcct gcaagaccta cccgccgtcc taaaatggcg cctgctatcc tgagacgccc    840
   gacatcacct gtgtctagag aatgcaatag tagtacggat agctgtgact ccggtccttc    900
   taacacacct cctgagatac acccggtggt cccgctgtgc cccattaaac cagttgccgt    960
   gagagttggt gggcgtcgcc aggctgtgga atgtatcgag gacttgctta acgagcctgg   1020
   gcaacctttg gacttgagct gtaaacgccc caggccataa aggcctaaat aaataatttt   1080
   tat                                                                 1083
<210> 10
   <211> 1588
   <212> DNA
   <213> artificial
<220>
   <223> expression cassette
<400> 10
   aaaaattgaa attttatttt ttttttttgg aatataaata aggaattcct gcaggctagc    60
   cccgggaagc ttccatggag cgaagaaacc catctgagcg gggggtacct gctggatttt   120
   ctggccatgc atctgtggag agcggttgtg agacacaaga atcgcctgct actgttgtct   180
   tccgtccgcc cggcgataat accgacggag gagcagcagc agcagcagga ggaagccagg   240
   cggcggcggc aggagcagag cccatggaac ccgagagccg gcctggaccc tcgggaatga   300
   atgttgtaca ggtggctgaa ctgtatccag aactgagacg cattttgaca attacagagg   360
   atgggcaggg gctaaagggg gtaaagaggg agcggggggc ttgtgaggct acagaggagg    420
   ctaggaatct agcttttagc ttaatgacca gacaccgtcc tgagtgtatt acttttcaac   480
   agatcaagga taattgcgct aatgagcttg atctgctggc gcagaagtat tccatagagc   540
   agctgaccac ttactggctg cagccagggg atgattttga ggaggctatt agggtatatg   600
   caaaggtggc acttaggcca gattgcaagt acaagatcag caaacttgta aatatcagga   660
   attgttgcta catttctggg aacggggccg aggtggagat agatacggag gatagggtgg   720
   cctttagatg tagcatgata aatatgtggc cgggggtgct tggcatggac ggggtggtta   780
   ttatgaatgt aaggtttact ggccccaatt ttagcggtac ggttttcctg gccaatacca   840
   accttatcct acacggtgta agcttctatg ggtttaacaa tacctgtgtg gaagcctgga   900
   ccgatgtaag ggttcggggc tgtgcctttt actgctgctg gaagggggtg gtgtgtcgcc   960
   ccaaaagcag ggcttcaatt aagaaatgcc tctttgaaag gtgtaccttg ggtatcctgt  1020
   ctgagggtaa ctccagggtg cgccacaatg tggcctccga ctgtggttgc ttcatgctag  1080
   tgaaaagcgt ggctgtgatt aagcataaca tggtatgtgg caactgcgag gacagggcct  1140
   ctcagatgct gacctgctcg gacggcaact gtcacctgct gaagaccatt cacgtagcca  1200
   gccactctcg caaggcctgg ccagtgtttg agcataacat actgacccgc tgttccttgc  1260
   atttgggtaa caggaggggg gtgttcctac cttaccaatg caatttgagt cacactaaga  1320
   tattgcttga gcccgagagc atgtccaagg tgaacctgaa cggggtgttt gacatgacca  1380
   tgaagatctg gaaggtgctg aggtacgatg agacccgcac caggtgcaga ccctgcgagt  1440
   gtggcggtaa acatattagg aaccagcctg tgatgctgga tgtgaccgag gagctgaggc  1500
   ccgatcactt ggtgctggcc tgcacccgcg ctgagtttgg ctctagcgat gaagatacag  1560
   attgaaggcc taaataaata atttttat                                     1588
<210> 11
   <211> 1687
   <212> DNA
   <213> artificial
<220>
   <223> expression cassette
<400> 11
   aaaaattgaa attttatttt ttttttttgg aatataaata aggaattcct gcaggctagc 60
   cccgggaagc ttccatggcc agtcgggaag aggagcagcg cgaaaccacc cccgagcgcg 120
   gacgcggtgc ggcgcgacgt cccccaacca tggaggacgt gtcgtccccg tccccgtcgc 180
   cgccgcctcc ccgggcgccc ccaaaaaagc ggatgaggcg gcgtatcgag tccgaggacg 240
   aggaagactc atcacaagac gcgctggtgc cgcgcacacc cagcccgcgg ccatcgacct 300
   cggcggcgga tttggccatt gcgcccaaga agaaaaagaa gcgcccttct cccaagcccg 360
   agcgcccgcc atcaccagag gtaatcgtgg acagcgagga agaaagagaa gatgtggcgc 420
   tacaaatggt gggtttcagc aacccaccgg tgctaatcaa gcatggcaaa ggaggtaagc 480
   gcacagtgcg gcggctgaat gaagacgacc cagtggcgcg tggtatgcgg acgcaagagg 540
   aagaggaaga gcccagcgaa gcggaaagtg aaattacggt gatgaacccg ctgagtgtgc 600
   cgatcgtgtc tgcgtgggag aagggcatgg aggctgcgcg cgcgctgatg gacaagtacc 660
   acgtggataa cgatctaaag gcgaacttca aactactgcc tgaccaagtg gaagctctgg 720
   cggccgtatg caagacctgg ctgaacgagg agcaccgcgg gttgcagctg accttcacca 780
   gcaacaagac ctttgtgacg atgatggggc gattcctgca ggcgtacctg cagtcgtttg 840
   cagaggtgac ctacaagcat cacgagccca cgggctgcgc gttgtggctg caccgctgcg 900
   ctgagatcga aggcgagctt aagtgtctac acggaagcat tatgataaat aaggagcacg 960
   tgattgaaat ggatgtgacg agcgaaaacg ggcagcgcgc gctgaaggag cagtctagca 1020
   aggccaagat cgtgaagaac cggtggggcc gaaatgtggt gcagatctcc aacaccgacg 1080
   caaggtgctg cgtgcacgac gcggcctgtc cggccaatca gttttccggc aagtcttgcg 1140
   gcatgttctt ctctgaaggc gcaaaggctc aggtggcttt taagcagatc aaggctttta 1200
   tgcaggcgct gtatcctaac gcccagaccg ggcacggtca ccttttgatg ccactacggt 1260
   gcgagtgcaa ctcaaagcct gggcacgcgc cctttttggg aaggcagcta ccaaagttga 1320
   ctccgttcgc cctgagcaac gcggaggacc tggacgcgga tctgatctcc gacaagagcg 1380
   tgctggccag cgtgcaccac ccggcgctga tagtgttcca gtgctgcaac cctgtgtatc 1440
   gcaactcgcg cgcgcagggc ggaggcccca actgcgactt caagatatcg gcgcccgacc 1500
   tgctaaacgc gttggtgatg gtgcgcagcc tgtggagtga aaacttcacc gagctgccgc 1560
   ggatggttgt gcctgagttt aagtggagca ctaaacacca gtatcgcaac gtgtccctgc 1620
   cagtggcgca tagcgatgcg cggcagaacc cctttgattt ttaaaggcct aaataaataa 1680
   tttttat 1687
<210> 12
   <211> 982
   <212> DNA
   <213> artificial
<220>
   <223> expression cassette
<400> 12
   aaaaattgaa attttatttt ttttttttgg aatataaata aggaattcct gcaggctagc      60
   cccgggaagc ttccatgact acgtccggcg ttccatttgg catgacacta cgaccaacac     120
   gatctcggtt gtctcggcgc actccgtaca gtagggatcg tctacctcct tttgagacag     180
   aaacccgcgc taccatactg gaggatcatc cgctgctgcc cgaatgtaac actttgacaa     240
   tgcacaacgt gagttacgtg cgaggtcttc cctgcagtgt gggatttacg ctgattcagg     300
   aatgggttgt tccctgggat atggttctaa cgcgggagga gcttgtaatc ctgaggaagt     360
   gtatgcacgt gtgcctgtgt tgtgccaaca ttgatatcat gacgagcatg atgatccatg     420
   gttacgagtc ctgggctctc cactgtcatt gttccagtcc cggttccctg cagtgtatag     480
   ccggcgggca ggttttggcc agctggttta ggatggtggt ggatggcgcc atgtttaatc     540
   agaggtttat atggtaccgg gaggtggtga attacaacat gccaaaagag gtaatgttta     600
   tgtccagcgt gtttatgagg ggtcgccact taatctacct gcgcttgtgg tatgatggcc     660
   acgtgggttc tgtggtcccc gccatgagct ttggatacag cgccttgcac tgtgggattt     720
   tgaacaatat tgtggtgctg tgctgcagtt actgtgctga tttaagtgag atcagggtgc     780
   gctgctgtgc ccggaggaca aggcgcctta tgctgcgggc ggtgcgaatc atcgctgagg     840
   agaccactgc catgttgtat tcctgcagga cggagcggcg gcggcagcag tttattcgcg     900
   cgctgctgca gcaccaccgc cctatcctga tgcacgatta tgactctacc cccatgtaga     960
   ggcctaaata aataattttt at                                              982
<210> 13
   <211> 3994
   <212> DNA
   <213> artificial
<220>
   <223> expression cassette
<400> 13
   aaaaattgaa attttatttt ttttttttgg aatataaata aggaattcct gcaggctagc     60
   cccgggaagc ttccatggcg tcggagaaca agcagcgccc cggctccccg ggccccaccg   120
   acgggccgcc gcccaccccg agcccagacc gcgacgagcg gggggccctc gggtggggcg   180
   cggagacgga ggagggcggg gacgaccccg accacgaccc cgaccacccc cacgacctcg   240
   acgacgcccg gcgggacggg agggcccccg cggcgggcac cgacgccggc gaggacgccg   300
   gggacgccgt ctcgccgcga cagctggctc tgctggcctc catggtagag gaggccgtcc   360
   ggacgatccc gacgcccgac cccgcggcct cgccgccccg gacccccgcc tttcgagccg   420
   acgacgatga cggggacgag tacgacgacg cagccgacgc cgccggcgac cgggccccgg   480
   cccggggccg cgaacgggag gccccgctac gcggcgcgta tccggacccc acggaccgcc   540
   tgtcgccgcg cccgccggcc cagccgccgc ggagacgtcg tcacggccgg tggcggccat   600
   cggcgtcatc gacctcgtcg gactccgggt cctcgtcctc gtcgtccgca tcctcttcgt   660
   cctcgtcgtc cgacgaggac gaggacgacg acggcaacga cgcggccgac cacgcacgcg   720
   aggcgcgggc cgtcgggcgg ggtccgtcga gcgcggcgcc ggcagccccc gggcggacgc   780
   cgcccccgcc cgggccaccc cccctctccg aggccgcgcc caagccccgg gcggcggcga   840
   ggacccccgc ggcctccgcg ggccgcatcg agcgccgccg ggcccgcgcg gcggtggccg   900
   gccgcgacgc cacgggccgc ttcacggccg ggcagccccg gcgggtcgag ctggacgccg   960
   acgcgacctc cggcgccttc tacgcgcgct atcgcgacgg gtacgtcagc ggggagccgt  1020
   ggcccggcgc cgggcccccg cccccggggc gggtgctgta cggcggcctg ggcgacagcc  1080
   gcccgggcct ctggggggcg cccgaggcgg aggaggcgcg acgccggttc gaggcctcgg  1140
   gcgccccggc ggccgtgtgg gcgcccgagc tgggcgacgc cgcgcagcag tacgccctga  1200
   tcacgcggct gctgtacacc ccggacgcgg aggccatggg gtggctccag aacccgcgcg  1260
   tggtccccgg ggacgtggcg ctggaccagg cctgcttccg gatctcgggc gccgcgcgca  1320
   acagcagctc cttcatcacc ggcagcgtgg cgcgggccgt gccccacctg ggctacgcca  1380
   tggcggccgg ccgcttcggc tggggcctgg cgcacgcggc ggccgccgtg gccatgagcc  1440
   gccgatacga ccgcgcgcag aagggcttcc tgctgaccag cctgcgccgc gcctacgcgc  1500
   ccctgttggc gcgcgagaac gcggcgctga cgggggccgc ggggagcccc ggcgccggcg  1560
   cagatgacga gggggtcgcc gccgtcgccg ccgccgcacc gggcgagcgc gcggtgcccg  1620
   ccgggtacgg cgccgcgggg atcctcgccg ccctggggcg gctgtccgcc gcgcccgcct  1680
   cccccgcggg gggcgacgac cccgacgccg cccgccacgc cgacgccgac gacgacgccg  1740
   ggcgccgcgc ccaggccggc cgcgtggccg tcgagtgcct ggccgcctgc cgcgggatcc  1800
   tggaggcgct ggccgagggc ttcgacggcg acctggcggc cgtcccgggg ctggccgggg  1860
   cccggcccgc cagccccccg cggccggagg gacccgcggg ccccgcttcc ccgccgccgc  1920
   cgcacgccga cgcgccccgc ctgcgcgcgt ggctgcgcga gctgcggttc gtgcgcgacg  1980
   cgctggtgct catgcgcctg cgcggggacc tgcgcgtggc cggcggcagc gaggccgccg  2040
   tggccgccgt gcgcgccgtg agcctggtcg ccggggccct gggccccgcg ctgccgcggg 2100
   acccgcgcct gccgagctcc gcggccgccg ccgccgcgga cctgctgttt gacaaccaga 2160
   gcctgcgccc cctgctggcg gcggcggcca gcgcaccgga cgccgccgac gcgctggcgg 2220
   ccgccgccgc ctccgccgcg ccgcgggagg ggcgcaagcg caagagtccc ggcccggccc 2280
   ggccgcccgg aggcggcggc ccgcgacccc cgaagacgaa gaagagcggc gcggacgccc 2340
   ccggctcgga cgcccgcgcc cccctccccg cgcccgcgcc cccctccacg cccccggggc 2400
   ccgagcccgc ccccgcccag cccgcggcgc cccgggccgc cgcggcgcag gcccgcccgc 2460
   gccccgtggc cgtgtcgcgc cggcccgccg agggccccga ccccctgggc ggctggcggc 2520
   ggcagccccc ggggcccagc cacacggcgg cgcccgcggc cgccgccctg gaggcctact 2580
   gctccccgcg cgccgtggcc gagctcacgg accacccgct gttccccgtc ccctggcgac 2640
   cggccctcat gtttgacccg cgggccctgg cctcgatcgc cgcgcggtgc gccgggcccg 2700
   cccccgccgc ccaggccgcg tgcggcggcg gcgacgacga cgataacccc cacccccacg 2760
   gggccgccgg gggccgcctc tttggccccc tgcgcgcctc gggcccgctg cgccgcatgg 2820
   cggcctggat gcgccagatc cccgaccccg aggacgtgcg cgtggtggtg ctgtactcgc 2880
   cgctgccggg cgaggacctg gccggcggcg gggcctcggg ggggccgccg gagtggtccg 2940
   ccgagcgcgg cgggctgtcc tgcctgctgg cggccctggc caaccggctg tgcgggccgg 3000
   acacggccgc ctgggcgggc aattggaccg gcgcccccga cgtgtcggcg ctgggcgcac 3060
   agggcgtgct gctgctgtcc acgcgggacc tggccttcgc cggggccgtg gagtttctgg 3120
   ggctgctcgc cagcgccggc gaccggcggc tcatcgtggt caacaccgtg cgcgcctgcg 3180
   actggcccgc cgacgggccc gcggtgtcgc ggcagcacgc ctacctggcg tgcgagctgc 3240
   tgcccgccgt gcagtgcgcc gtgcgctggc cggcggcgcg ggacctgcgc cgcacggtgc 3300
   tggcctcggg ccgcgtgttc ggcccggggg tcttcgcgcg cgtggaggcc gcgcacgcgc 3360
   gcctgtaccc cgacgcgccg ccgctgcgcc tgtgccgcgg cggcaacgtg cgctaccgcg 3420
   tgcgcacgcg cttcggcccg gacacgccgg tgcccatgtc cccgcgcgag taccgccggg 3480
   ccgtgctgcc ggcgctggac ggccgggcgg cggcctcggg gaccaccgac gccatggcgc 3540
   ccggcgcgcc ggacttctgc gaggaggagg cccactcgca cgccgcctgc gcgcgctggg 3600
   gcctgggcgc gccgctgcgg cccgtgtacg tggcgctggg gcgcgaggcg gtgcgcgccg 3660
   gcccggcccg gtggcgcggg ccgcggaggg acttttgcgc ccgcgccctg ctggagcccg 3720
   acgacgacgc ccccccgctg gtgctgcgcg gcgacgacga cggcccgggg gccctgccgc 3780
   cggcgccgcc cgggattcgc tgggcctcgg ccacgggccg cagcggcacc gtgctggcgg 3840
   cggcgggggc cgtggaggtg ctgggggcgg aggcgggctt ggccacgccc ccgcggcggg 3900
   aagttgtgga ctgggaaggc gcctgggacg aagacgacgg cggcgcgttc gagggggacg 3960
   gggtgctgta aaggcctaaa taaataattt ttat 3994
<210> 14
   <211> 1636
   <212> DNA
   <213> artificial
<220>
   <223> expression cassette
<400> 14
   aaaaattgaa atttattttt ttttttttgg aatataaata aggaattcct gcaggctagc      60
   cccgggaagc ttccatggcg actgacattg atatgctaat tgacctcggc ctggacctct   120
   ccgacagcga tctggacgag gacccccccg agccggcgga gagccgccgc gacgacctgg   180
   aatcggacag cagcggggag tgttcctcgt cggacgagga catggaagac ccccacggag   240
   aggacggacc ggagccgata ctcgacgccg ctcgcccggc ggtccgcccg tctcgtccag   300
   aagaccccgg cgtacccagc acccagacgc ctcgtccgac ggagcggcag ggccccaacg   360
   atcctcaacc agcgccccac agtgtgtggt cgcgcctcgg ggcccggcga ccgtcttgct   420
   cccccgagca gcacgggggc aaggtggccc gcctccaacc cccaccgacc aaagcccagc   480
   ctgcccgcgg cggacgccgt gggcgtcgca ggggtcgggg tcgcggtggt cccggggctg   540
   ccgatggttt gtcggacccc cgccggcgtg cccccagaac caatcgcaac cctgggggac   600
   cccgccccgg ggcggggtgg acggacggcc ccggcgcccc ccatggcgag gcgtggcgcg   660
   gcagtgagca gcccgaccca cccggaggcc agcggacacg gggcgtgcgc caagcacccc   720
   ccccgctaat gacgctggcg attgcccccc cgcccgcgga cccccgcgcc ccggccccgg   780
   agcgaaaggc gcccgccgcc gacaccatcg acgccaccac gcggttggtc ctgcgctcca   840
   tctccgagcg cgcggcggtc gaccgcatca gcgagagctt tggccgcagc gcacaggtca   900
   tgcacgaccc ctttgggggg cagccgtttc ccgccgcgaa tagcccctgg gccccggtgc   960
   tggcgggcca aggagggccc tttgacgccg agaccagacg ggtctcctgg gaaaccttgg  1020
   tcgcccacgg cccgagcctc tatcgcactt ttgccggcaa tcctcgggcc gcatcgaccg  1080
   ccaaggccat gcgcgactgc gtgctgcgcc aagaaaattt catcgaggcg ctggcctccg  1140
   ccgacgagac gctggcgtgg tgcaagatgt gcatccacca caacctgccg ctgcgccccc  1200
   aggaccccat tatcgggacg accgcggctg tgctggataa cctcgccacg cgcctgcggc  1260
   cctttctcca gtgctacctg aaggcgcgag gcctgtgcgg cctggacgaa ctgtgttcgc  1320
   ggcggcgtct ggcggacatt aaggacattg catccttcgt gtttgtcatt ctggccaggc  1380
   tcgccaaccg cgtcgagcgt ggcgtcgcgg agatcgacta cgcgaccctt ggtgtcgggg  1440
   tcggagagaa gatgcatttc tacctccccg gggcctgcat ggcgggcctg atcgaaatcc  1500
   tagacacgca ccgccaggag tgttcgagtc gtgtctgcga gttgacggcc agtcacatcg  1560
   tcgccccccc gtacgtgcac ggcaaatatt tttattgcaa ctccctgttt tagaggccta  1620
   aataaataat ttttat                                                  1636